# EUROPEAN PATENT SPECIFICATION

(11) **EP 2 836 607 B1**
(45) Date of publication and mention of the grant of the patent: **22.11.2017**
(21) Application number: 13772909.1
(22) Date of filing: 05.04.2013
(51) Int. Cl.: C12Q 1/68, C07K 16/00

(54) **DETECTION OF IMMUNOGLOBULIN LIGHT CHAIN RESTRICATION BY RNA IN SITU HYBRIDIZATION**
ERKENNUNG VON RESTRIKTIONEN LEICHTER IMMUNOGLOBULINKETTEN DURCH IN-SITU-RNA-HYBRIDISIERUNG
DÉTECTION D'UNE RESTRICTION DE CHAÎNE LÉGÈRE D'IMMUNOGLOBULINE PAR HYBRIDATION IN SITU D'ARN

(30) Priority: 05.04.2012 US 201261620634 P; 22.10.2012 US 201261717064 P; 15.03.2013 US 201313843408
(43) Date of publication of application: 18.02.2015
(73) Proprietor: Advanced Cell Diagnostics, Inc., Newark, CA 94560 (US); THE CLEVELAND CLINIC FOUNDATION, Cleveland, OH 44195 (US)
(72) Inventor: MA, Xiao-Jun, Hayward, CA 94545 (US); LUO, Yuling, Hayward, CA 94545 (US); TUBBS, Raymond, Cleveland, OH 44195 (US)
(74) Representative: Jones Day
(86) International application number: PCT/US2013/035460
(87) International publication number: WO 2013/152295

(56) References cited:
- WO-A1-00/67021
- US-A1- 2012 040 858
- LANG: "Demonstration of Kappa and Lambda Light Chains by Dual Chromogenic In Situ Hybridization of Formalin-Fixed, Acid-Decalcified, and Paraffin-Embedded Bone Marrow Trephine Biopsies", JOURNAL OF HISTOTECHNOLOGY, DOWNERS GROVE, IL, US, vol. 33, no. 1, 1 March 2010 (2010-03-01), pages 9-13, XP008175060, ISSN: 0147-8885
- BECK ET AL: "Automated Colorimetric In Situ Hybridization (CISH) Detection of Immunoglobulin (Ig) Light Chain mRNA Expression in Plasma Cell (PC) Dyscrasias and Non-Hodgkin Lymphoma", DIAGNOSTIC MOLECULAR PATHOLOGY, LIPPINCOTT WILLIAMS AND WILKINS, US, vol. 12, no. 1, 1 March 2003 (2003-03-01), pages 14-20, XP008175061, ISSN: 1052-9551
- PRINGLE J H ET AL: "IN SITU HYBRIDIZATION OF IMMUNOGLOBULIN LIGHT CHAIN MRNA IN PARAFFIN SECTIONS USING BIOTINYLATED OR HAPTEN-LABELLED OLIGONUCLEOTIDE PROBES", JOURNAL OF PATHOLOGY, JOHN WILEY & SONS LTD, GB, vol. 162, no. 3, 1 January 1990 (1990-01-01), pages 197-207, XP009003896, ISSN: 0022-3417, DOI: 10.1002/PATH.1711620305
- MCNICOL A M ET AL: "COMPARISON OF IN SITU HYBRIDISATION AND POLYMERASE CHAIN REACTION IN THE DIAGNOSIS OF B CELL LYMPHOMA", JOURNAL OF CLINICAL PATHOLOGY, BMJ PUBLISHING GROUP, GB, vol. 51, no. 3, 1 March 1998 (1998-03-01), pages 229-233, XP009012567, ISSN: 0021-9746, DOI: 10.1136/JCP.51.3.229
- MARIA LIBERA ASCIERTO ET AL: "A signature of immune function genes associated with recurrence-free survival in breast cancer patients", BREAST CANCER RESEARCH AND TREATMENT, KLUWER ACADEMIC PUBLISHERS, BO, vol. 131, no. 3, 11 April 2011 (2011-04-11), pages 871-880, XP035004941, ISSN: 1573-7217, DOI: 10.1007/S10549-011-1470-X
- R. R. TUBBS ET AL: "Ultrasensitive RNA In Situ Hybridization for Detection of Restricted Clonal Expression of Low-Abundance Immunoglobulin Light Chain mRNA in B-Cell Lymphoproliferative Disorders", AMERICAN JOURNAL OF CLINICAL PATHOLOGY, vol. 140, no. 5, 11 October 2013 (2013-10-11), pages 736-746, XP055191951, ISSN: 0002-9173, DOI: 10.1309/AJCPJTWK07FSABRJ
- LANG.: 'Demonstration of Kappa and Lambda Light Chains by Dual Chromogenic In Situ Hybridization of Formalin-Fixed, Acid-Decalcified, and Paraffin-Embedded Bone Marrow Trephine Biopsies' THE JOURNAL OF HISTOTECHNOLOGY vol. 33, no. 1, 01 March 2010, pages 9 - 13, XP008175060
- BECK ET AL.: 'Automated Colorimetric In Situ Hybridization (CISH) Detection of Immunoglobulin (Ig) Light Chain mRNA Expression in Plasma Cell (PC) Dyscrasias and Non-Hodgkin Lymphoma' DIAGNOSTIC MOLECULAR PATHOLOGY vol. 12, no. 1, 01 March 2003, pages 14 - 20, XP008175061
- MCNICOL ET AL.: 'Comparison of in situ hybridisation and polymerase chain reaction in the diagnosis of B cell lymphoma' JOURNAL OF CLINICAL PATHOLOGY vol. 51, no. 229-23, 01 March 1998, XP009012567
- PRINGLE ET AL.: 'In Situ Hybridization of Immunoglobulin Light Chain mRNA in Paraffin Sections Using Biotinylated or Hapten-Labelled Oligonucleotide Probes' JOURNAL OF PATHOLOGY vol. 162, 01 November 1990, pages 197 - 207, XP009003896

## Description

This application claims the benefit of priority of United States application serial No. 13/843,408, filed March 15, 2013, and the benefit of priority of United States Provisional application serial No. 61/620,634, filed April 5, 2012, and the benefit of priority of United States Provisional application serial No. 61/717,064, filed October 22, 2012.

### BACKGROUND OF THE INVENTION

The present invention relates generally to *in situ* hybridization assays, and more specifically to assays for diagnosing B cell neoplasms.

Immunoglobulins are composed of two heavy and two light chains. During B cell differentiation, DNA encoding immunoglobulin genes undergoes rearrangement to create unique combinations of immunoglobulin domains that result in a large diversity of possible immunoglobulins available to the immune system of an individual. Each mature B cell expresses a distinct combination of immunoglobulin domains. There are two types of light chains, designated κ (kappa) and λ (lambda). During B cell maturation, the DNA rearrangements that occur result in each mature B cell expressing an immunoglobulin having either a kappa light chain or a lambda light chain. Thus, within a given differentiated B cell, the B cell will express either a kappa light chain or a lambda chain. In a normal mammalian organism, the organism will have a population of B cells that individually express either kappa or lambda light chains but as a population will have a mixture of kappa and lambda light chain expressing cells. Such a population of cells is considered to be polyclonal.

Neoplastic cells are characterized by unregulated cell growth. In the case of a B cell neoplasm, the unregulated cell growth of a neoplastic B cell results in a population B cells that include many copies of the same cell. Since a mature B cell generally expresses either kappa or lambda light chains, a population of neoplastic B cells will exhibit the characteristic of many copies of the same cell and therefore many copies of cells expressing only kappa chain or only lambda light chain. Such a population containing multiple copies of the same cell is considered to be clonal, and clonality is characteristic of the aberrant cell growth of a neoplastic cell.

Clonal expansion is therefore one of the hallmarks of B cell malignancies, and is most commonly manifested as restricted expression of immunoglobulin (Ig) light chain (either kappa or lambda)(Cook, "Molecular Hematopathology," Tubbs & Stoler, eds., in Cell and Tissue Based Molecular Pathology, Churchill Livingstone Elsevier (2008)). Clinical laboratory detection of Ig light chain restriction (LCR) is a helpful ancillary tool in the differential diagnosis that includes lymphoid hyperplasia, atypical lymphoid hyperplasia, chronic inflammation, and B cell neoplasms (Cook, *supra*, 2008; Taylor, Applied Immunohistochem. Mol. Morphology 17:470-482 (2009a); Taylor, Applied Immunohistochem. Mol. Morphology 17:366-374 (2009b)).

Demonstration of Kappa and Lambda light chains by dual chromogenic *in situ* hybridization of formalin-fixed, acid-decalcified, and paraffin-embedded bone marrow trephine biopsies was already shown in the prior art (Lang, Journal of Histotechnology 33:9-13 (2010)) but without detection of IGLL5.

LCR identification by immunohistochemistry and conventional chromogenic *in situ* hybridization (CISH) is feasible for B cell neoplasms that express abundant kappa or lambda mRNA and cytoplasmic immunoglobulin protein (Beck et al., Diagnostic Mol. Pathol. 12:14-20 (2003)). Plasma cell myeloma LCR is almost always feasible in formalin fixed paraffin embeded (FFPE) tissue. But only uncommon subsets of non-Hodgkin lymphomas (NHL), the few with abundant Ig mRNA and protein expression, are evaluable by these traditional methods (Beck et al., *supra*, 2003). Conversely, autoradiographic *in situ* hybridization is sufficiently sensitive to detect the very low levels of light chain mRNA in a very high percentage of NHL of all subtypes (Segal et al., Diagnostic Mol. Pathol.: Am. J. Surg. Pathol., part B, 3:170-177 (1994)), including the most common NHL variants - follicular lymphoma, diffuse large B cell lymphoma, small B lymphocytic lymphoma, and extranodal marginal zone lymphoma of MALT type. But two weeks or more are required for the autoradiographic signals to develop (Segal et al., *supra*, 1994), a delay that is unacceptable for use in clinical management.

Thus, there exists a need for accurate and rapid assays for the identification of benign and cancerous B cell samples. The present invention satisfies this need and provides related advantages as well.

### SUMMARY OF INVENTION

The invention provides a method for detecting immunoglobulin light chain restriction and clonality in B cells. The method can include the steps of obtaining a sample of B cells from a subject; conducting one or more *in situ* hybridization assays on the sample using (i) at least one probe set which is designed to specifically hybridize to immunoglobulin kappa chain constant region (IGKCR) RNA; (ii) at least one probe set which is designed to specifically hybridize to immunoglobulin lambda chain constant region (IGLCR) RNA; and optionally (iii) at least one probe set which is designed to specifically hybridize to immunoglobulin lambda-like polypeptide 5 (IGLL5) RNA; detecting signal associated with hybridized IGKCR probe, signal associated with hybridized IGLCR probe, and signal associated with hybridized IGLL5 probe in a population of B cells in the sample; and determining a pattern of signal associated with hybridized IGKCR probe, hybridized IGLCR probe, and hybridized IGLL5 probe within individual cells in the B cell population, wherein the pattern of signal within individual cells indicates the presence or absence of light chain restriction and clonality of the B cells.

### BRIEF DESCRIPTION OF THE DRAWINGS

The patent or application file contains at least one drawing executed in color. Copies of this patent or patent application publication with color drawings will be provided by the Office upon request and payment of the necessary fee.
Figures 1A and 1B show examples of an *in situ* hybridization assay exhibiting clear restricted light chain expression in kappa-restricted (Pattern 1, Figure 1A, left panel) and lambda-restricted (Pattern 2, Figure 1B, right panel) lymphomas, where one of the two light chains was expressed in the tumor cells.
Figure 2 shows an *in situ* hybridization assay, in which both kappa (left panel) and lambda (right panel) signals were detected.
Figures 3A-3C show an *in situ* hybridization assay, in which both kappa and lambda signals were detected. Figure 3A shows kappa staining, and Figure 3B shows lambda staining. Figure 3C shows treatment with RNase A and use of a lambda probe.
Figure 4 shows a schematic diagram of a portion of human chromosome 22 in a region encoding the Ig lambda light chain.
Figures 5A-5D show staining of tissue sections with lambda and IGLL5 probes. Figures 5A and 5B show staining of adjacent tissue sections with lambda and IGLL5 probes, respectively. Figures 5C and 5D show a duplex assay using lambda and IGLL5 probes detected by fluorescence or a chromogenic assay, respectively.
Figure 6 shows an *in situ* hybridization assay of B cell lymphoma, where both kappa and lambda signals were present in the same cells. Tissue samples were stained with kappa (upper left panel), lambda (upper right panel), IGLL5 (lower left panel), or duplex stained with kappa (DAB, brown staining) and lambda (Fast Red, red staining)(lower right panel).
Figure 7 shows an *in situ* hybridization assay showing kappa and lambda signals in different cells. Duplex staining was performed with kappa (stained with Deep Space Black, black staining) and lambda (Fast Red, red staining) probes.
Figure 8 shows a schematic for determining light chain restriction based on patterns of kappa, lambda and IGLL5 mRNA expression. Numbers 1-5 correspond to 5 observed patterns of expression.
Figure 9 shows exemplary probe configurations for detecting target nucleic acids such as immunglobulin kappa light chain constant region (IGKCR) mRNA, immunoglobulin lambda chain constant region (IGLCR) mRNA or immunoglobulin lambda-like polypeptide 5 (IGLL5).

### DETAILED DESCRIPTION OF THE INVENTION

The present invention relates to methods for detecting immunoglobulin light chain restriction and clonality of B cells. The methods of the invention provide highly sensitive assays that are useful for identifying neoplastic B cells. The methods of the invention can also be used to distinguish and identify neoplastic B cells that would generally have been characterized as benign using traditional immunoglobulin light chain restriction analysis. The methods of the invention can therefore be used in diagnostic applications to provide greater confidence in the validity of diagnostic assays for the assessment of B cell neoplasms.

An RNA ISH technology platform called RNAscope® has been developed (Advanced Cell Diagnostics, Inc.; Hayward CA), which has single-molecule sensitivity, is capable of multiplex detection, and is compatible with formalin fixed and paraffin embedded (FFPE) tissue (see, for example, Masand et al., 5:108-116 (2011); Ukpo et al., Am. J. Surg. Pathol. 35:1343 (2011); Wang et al., J. Mol. Diagnostics 14:22-29 (2012)). RNAscope® represents a fundamental technological advance in molecular morphology and provides for numerous applications in diagnostic pathology, especially those tissue section-based assays which must detect very low levels of mRNA. As disclosed herein, RNAscope® was utilized for light chain restriction (LCR) detection in B cells and was found to provide superior results and sensitivity over previous methods of LCR analysis. As further disclosed herein, application of RNAscope® to LCR detection yielded a more complex staining pattern than simple kappa and lambda chain restriction. Analysis of these patterns allowed the development of an assay and an interpretation algorithm for accurately determining LCR, and thus clonality of B cells (see Example).

Neoplasms of the immune system are a heterogeneous group of tumors of various cell origins. Non-Hodgkin's lymphomas (NHL) are the single largest group of neoplasms of the immune system. In the case of B cell neoplasms, the characteristics of the neoplasm depend on the type of neoplasm, including the stage of B cell development associated with the neoplasm. The cell of origin of the neoplasm can be associated with a stage of B cell development and is generally a medullary B cell, follicular B cell or immunoblastic B cell (see Cecil Textbook of Medicine, 20th ed., Bennet and Plum, eds., WB Saunders, Philadelphia PA (1996); Harrison's Principles of Internal Medicine, 14th ed., Fauci et al., eds., McGraw-Hill, New York (1998)). B cell lymphomas can be classified based on the grade (according to 5-year survival) and include, for example, low-grade lymphomas such as small lymphocytic lymphoma (SLL), follicular, small cleaved cell lymphoma (FSCL), follicular, mixed small cleaved and large cell lymphoma (FML), and mucosa-associated lymphoid tissue (MALT); intermediate-grade lymphomas such as follicular, large cell lymphoma (FLCL), diffuse, small cleaved cell lymphoma (DSCL), diffuse, mixed small cleaved and large cell lymphoma (DML), diffuse, large cell (cleaved and noncleaved) lymphoma (DLCL); and high-grade lymphomas such as large cell immunoblastic lymphoma (IBL), and small noncleaved cell (Burkitt and non-Burkitt) lymphoma (SNCL).

Establishing clonality of B lymphocyte proliferation has traditionally been accomplished by the demonstration of a single class of heavy and/or light chain cell surface immunoglobulin. At the DNA level, clonality can be confirmed by the presence of a single immunoglobulin gene rearrangment. In precursor B lymphocyte neoplasms, which lack surface immunoglobulins, gene rearrangement studies have been necessary to demonstrate clonality. Traditional assay methods for establishing clonality have included flow cytometry and/or immunohistochemistry based on surface expression of immunoglobulins or other cell surface markers or nucleic acid based detection assays such as *in situ* hybridization or polymerase chain reaction (PCR) assays. As discussed herein, one hallmark of clonality of B lymphocytes is restriction to expression of either kappa or lambda light chains. The present invention relates to the use of an exquisitely sensitive *in situ* hybridization assay that permits highly accurate and rapid detection of immunoglobulin light chain restriction, which can be used in the diagnosis of B cell neoplasms.

The assays of the invention are useful in clinical applications, particularly in neoplasms of B cells. Accurate determination of LCR and B cell clonality by the assay and alogorithm of the invention, as described herein, are useful for diagnosing B cell proliferative diseases, allowing discrimination of B cell neoplasms (clonal B cell populations) from benign B cell proliferative diseases (polyclonal). The methods of the invention are particularly beneficial in distinguishing a sample containing apparent polyclonal B cell staining patterns that would traditionally be identified as negative or indeterminate from a sample containing cells having both kappa and lambda staining that are monoclonal, that is, neoplastic.

Further, the invention additionally provides assays and algorithms for discrimination of previously unrecognized patterns of various kappa/lambda/IGLL5 expression that has potential clinical significance. It has been shown that dual light chain protein expression as determined by flow cytometry is associated with aggressive B cell lymphoma (Fujiwara et al., Internal Med. 46:1458-1461 ((2007)). It is possible that the different patterns of kappa/lambda/IGLL5 mRNA expression are biomarkers of different subtypes of B cell lymphoma that may have distinct prognosis and/or response to therapies. Thus, the methods of the invention can be used to identify subtypes of B cell lymphomas and/or to predict or monitor response to B cell lymphoma therapies.

As used herein, the term "nucleic acid," or alternatively the term "polynucleotide," refers to a polymer of nucleotide monomer units, as is well known to those skilled in the art. Exemplary nucleic acids include, for example, DNA and RNA, including mRNA, siRNA, hnRNA, genomic DNA, cDNA, or other well known forms of nucleic acids. The nucleic acid or polynucleotide can contain naturally occurring nucleotides, including well known naturally occurring nucleotide modifications, as well as non-naturally occurring nucleotides such as those made by chemical synthesis. Such modifications include, but are not limited to, peptide nucleic acids (PNAs), modified oligonucleotides, for example, oligonucleotides comprising nucleotides that are not typical to biological RNA or DNA, such as 2'-O-methylated oligonucleotides, and the like. The nucleotides of the polynucleotide can be deoxyribonucleotides, ribonucleotides or nucleotide analogs, can be natural or non-natural, and can be unsubstituted, unmodified, substituted or modified. The nucleotides can be linked by phosphodiester bonds, or by phosphorothioate linkages, methylphosphonate linkages, boranophosphate linkages, or the like. The polynucleotide can additionally comprise non-nucleotide elements such as labels, quenchers, blocking groups, or the like, as disclosed herein. The polynucleotide can be single-stranded or double-stranded.

As used herein, a "nucleic acid target" or "target nucleic acid" refers to a nucleic acid, or a region thereof, that is intended to be detected. One skilled in the art can readily determine target nucleic acids desired to be detected by methods of the invention. As describe herein, the invention relates to methods of detecting immunoglobulin light chain restriction and clonality in B cells. In the methods of the invention, the target nucleic acid is generally immunoglobulin kappa chain constant region (IGKCR) mRNA, immunoglobulin lambda chain constant region (IGLCR) mRNA and immunoglobulin lambda-like polypeptide 5 (IGLL5). One skilled in the art can readily design probes suitable for detecting IGKCR, IGLCR and IGLL5 using well known methods of probe design and sequences available in public databases. Exemplary IGKCR sequences can be found in GenBank accession No. AF026381.1 (GI:3169769). Exemplary IGLCR sequences can be found in GenBank accession Nos U07991.1 (GI:468246) and HF541912.1 (GI:444737700). Exemplary IGLL5 sequences can be found in GenBank accession Nos. NM_001178126.1 (GI:295986607) and NM_0012S6296.1 (GI:372466585). Other exemplary sequences of IGKCR, IGLCR and/or IGLL5 are available in public databases such as GenBank at the NCBI or EMBL and can also be used. If desired, the probes can take into account DNA rearrangements that occur during B cell development, including the selection of probes in particular exons.

As used herein, a "label" is a moiety that facilitates detection of a molecule. Common labels in the context of the present invention include fluorescent, luminescent, light-scattering, and/or colorimetric labels. Suitable labels include enzymes, and fluorescent and chromogenic moieties, as well as radionuclides, substrates, cofactors, inhibitors, chemiluminescent moieties, magnetic particles, and the like. In a particular embodiment of the invention, the label is an enzyme. Exemplary enzyme labels include, but are not limited to Horse Radish Peroxidase (HRP), Alkaline Phosphatase (AP), β-galactosidase, glucose oxidase, and the like, as well as various proteases. Other labels include, but are not limited to, fluorophores, Dinitrophenyl (DNP), and the like. Labels are well known to those skilled in the art, as described, for example, in Hermanson, Bioconjugate Techniques, Academic Press, San Diego (1996), and U.S. Patent Nos. 3,817,837; 3,850,752; 3,939,350; 3,996,345; 4,277,437; 4,275,149; and 4,366,241. Many labels are commercially available and can be used in methods and assays of the invention, including detectable enzyme/substrate combinations (Pierce, Rockford IL; Santa Cruz Biotechnology, Dallas TX; Invitrogen, Carlsbad CA). In a particular embodiment of the invention, the enzyme can utilize a chromogenic or fluorogenic substrate to produce a detectable signal, as described herein.

As used herein, "*in situ* hybridization" or "ISH" refers to a type of hybridization that uses a directly or indirectly labeled complementary DNA or RNA strand, such as a probe, to bind to and localize a specific nucleic acid, such as DNA or RNA, in a sample, in particular a portion or section of tissue (*in situ*). The probe types can be double stranded DNA (dsDNA), single stranded DNA (ssDNA), single stranded complimentary RNA (sscRNA), messenger RNA (mRNA), micro RNA (miRNA), and/or synthetic oligonucleotides. The term "fluorescent *in situ* hybridization" or "FISH" refers to a type of ISH utilizing a fluorescent label. The term "chromogenic *in situ* hybridization" or "CISH" refers to a type of ISH with a chromogenic label. ISH, FISH and CISH methods are well known to those skilled in the art (see, for example, Stoler, Clinics in Laboratory Medicine 10(1):215-236 (1990); *In situ hybridization.* A practical approach, Wilkinson, ed., IRL Press, Oxford (1992); Schwarzacher and Heslop-Harrison, Practical in situ hybridization, BIOS Scientific Publishers Ltd, Oxford (2000)).

As used herein, the phrase "immunoglobulin light chain restriction" refers to the expression of kappa or lambda light chain in a B cell. As a B cell matures and undergoes DNA rearrangements to produce a particular immunoglobulin, the expression of the light chain becomes restricted to either kappa or lambda but not both. Immunoglobulin light chain restriction is well known to those skilled in the art, particularly in the area of hematopathology and diagnosis of B cell neoplasms (see, for example, Knowles, Neoplastic Hematopathology, 2nd edl., Lippincott Williams & Wilkins, Philadelphia PA (2001); Lehmen et al., Am. J. Pathol. 159:2023-2029 (2001); Arber, J. Mol. Diagnsotics 2:178-190 (2000); Plank et al., Am. J. Clin. Pathol. 103:330-337 (1995)). LCR is a widely recognized hallmark of B cell lymphomas, but the existence of non-restricted light chain expression, that is, expression of both kappa and lambda chains in the same B cells, has been reported in rare cases of B cell neoplasms (Xu, Arch. Pathol. Lab. Med. 130:853-856 (2006).

As used herein, the term "clone" refers to a group of cells produced by cell division from a single cell. As used herein, the term "clonality," when used in reference to a B cell, refers to a population of B cells produced by cell division from a parental B cell. The detection of clonality is a standard tool in the determination of neoplastic B cells in the area of hematopathology. The detection of B cell clonality is well known to those skilled in the art, particularly in the area of hematopathology and diagnosis of B cell neoplasms (see, for example, Knowles, Neoplastic Hematopathology, 2nd edl., Lippincott Williams & Wilkins, Philadelphia PA (2001); Lehmen et al., Am. J. Pathol. 159:2023-2029 (2001); Arber, J. Mol. Diagnsotics 2:178-190 (2000); Plank et al., Am. J. Clin. Pathol. 103:330-337 (1995)).

As used herein, the phrase "pattern of signal" refers to an observable characteristic in a population of cells as measured by a detectable signal. In aspects of the invention where nucleic acids are detected, the signal is associated with a nucleic acid detection method, for example, a label as disclosed herein. As described herein, measurable and distinct patterns of signal within a population of cells can be used to correlate with a normal or neoplastic phenotype of a B cell population.

In one embodiment, the invention provides a method for detecting immunoglobulin light chain restriction and clonality in B cells. The method can include the steps of obtaining a sample of B cells from a subject; conducting a duplex *in situ* hybridization assay on the sample using (i) at least one probe set which is designed to specifically hybridize to immunoglobulin kappa chain constant region (IGKCR) RNA; and (ii) at least one probe set which is designed to specifically hybridize to immunoglobulin lambda chain constant region (IGLCR) RNA; detecting signal associated with hybridized IGKCR probe and signal associated with hybridized IGLCR probe in a population of B cells in the sample; and determining a pattern of signal associated with hybridized IGKCR probe and hybridized IGLCR probe within individual cells in the B cell population, wherein the pattern of signal within individual cells indicates the presence or absence of light chain restriction and clonality of the B cells.

As disclosed herein, the methods of the invention utilize a highly sensitive *in situ* assay to detect immunoglobulin light chain restriction and clonality of B cells. Due to the high sensitivity of the assay, IGKCR and IGLCR expression can be measured at the level of a single nucleic acid molecule and at the spatial resolution of an individual cell. The assays are therefore applicable in identifying clonality of B cells that might otherwise have been overlooked and categorized as benign and are particularly useful in diagnostic applications. As disclosed herein, the pattern of signal associated with IGKCR and IGLCR probes, as well as with IGLL5 probes, has been determined to indicate the presence of light chain restriction and clonality of the B cells in a sample.

The pattern in individual B cells of only signal associated with hybridized IGKCR probe indicates kappa restriction in the sample. While this pattern was previously understood to indicate kappa restriction, the methods of the invention provide highly sensitive detection down to the level of a single nucleic acid molecule and at the spatial resolution of an individual cell. The pattern in individual B cells of only signal associated with hybridized IGLCR probe indicates lambda restriction in the sample. Similarly, while this pattern was previously understood to indicate lambda restriction, the methods of the invention provide highly sensitive detection down to the level of a single nucleic acid molecule and at the spatial resolution of an individual cell.

The pattern of detecting both signal associated with hybridized IGKCR probe and signal associated with hybridized IGLCR probe in separate B cells within the population indicates the sample is polyclonal. While this pattern was previously understood to indicate polyclonality, the methods of the invention provide highly sensitive detection down to the level of a single nucleic acid molecule and at the spatial resolution of an individual cell. The pattern of both signal associated with hybridized IGKCR probe and signal associated with hybridized IGLCR probe in the same B cell indicates the sample is monoclonal. Again, the method of the invention provides the ability to detect patterns of expression at the level of a single nucleic acid molecule and at the spatial resolution of an individual cell. Previous methods would not have been able to do so, and in the situation where both kappa and lambda probes are detected in a sample, such a sample likely would have been indicated as a negative, polyclonal cell sample because detection of both kappa and lambda light chain expression in a sample is a signature of polyclonality, that is, a normal B cell sample. However, it was surprisingly discovered using the very sensitive assays of the invention that a cell population with a pattern of positive signals for both lambda and kappa light chain mRNA expression can in fact be clonal. The methods of the invention are therefore able to discriminate such a false negative and correctly identify the sample as containing a clonal B cell population. Thus, the invention provides methods that permit more accurate diagnosis and significant elimination of false negatives or ambiguities. Such a surprising observation can be applied clinically to eliminate false negatives and resolve ambiguities due to the very sensitive assays of the invention, which can measure signals associated with kappa and lambda probes at the level of a single nucleic acid molecule and at the spatial resolution of an individual cell. Such methods are particularly useful in diagnosing human B cell neoplasms.

The invention provides a method for detecting immunoglobulin light chain restriction and clonality in B cells. Such a method can include the steps of obtaining a sample of B cells from a subject; conducting one or more *in situ* hybridization assays on the sample using (i) at least one probe set which is designed to specifically hybridize to immunoglobulin kappa chain constant region (IGKCR) RNA; (ii) at least one probe set which is designed to specifically hybridize to immunoglobulin lambda chain constant region (IGLCR) RNA; and (iii) at least one probe set which is designed to specifically hybridize to immunoglobulin lambda-like polypeptide 5 (IGLL5) RNA; detecting signal associated with hybridized IGKCR probe, signal associated with hybridized IGLCR probe, and signal associated with hybridized IGLL5 probe in a population of B cells in the sample; and determining a pattern of signal associated with hybridized IGKCR probe, hybridized IGLCR probe, and hybridized IGLL5 probe within individual cells in the B cell population, wherein the pattern of signal within individual cells indicates the presence or absence of light chain restriction and clonality of the B cells.

In one embodiment of a method of the invention, the one or more *in situ* hybridization assays can be conducted as singleplex reactions on serial sections. In such a case, the IGKCR, IGLCR and IGLL5 probes are used in separate *in situ* hybridization assays and the associated signals are measured independently, generally on serial sections. Alternatively, the hybridization of the IGKCR probe set and the IGLCR probe set can be hybridized in the same *in situ* hybridization assay. Although it is optionally possible to perform a triplex assay including the IGLL5 probe set in the same hybridization assay, generally hybridization of the IGLL5 probe set is performed in a separate *in situ* hybridization assay from the assay in which the IGKCR probe set and the IGLCR probe set are hybridized.

As is well known in the art, appropriate negative controls can optionally be included in a hybridization assay. For example, a hybridization assay can be conducted using at least one negative control probe set. The use and design of negative controls for use in an *in situ* hybridization assay are well known to those skilled in the art. Particularly useful negative controls include those that would not hybridize in particular to a B cell being characterized. Such a negative control can therefore be based on probes that hybridize to sequences known not to be expressed in a B cell, for example, tissue specific probes. A particularly useful negative control would be a probe that is designed for a completely different organism, in particular a non-mammalian organism, and more particularly a non-eukaryotic organism such as a bacterium. One skilled in the art will readily know how to select appropriate negative controls suitable for an *in situ* hybridization assay.

In a method of the invention utilizing IGKCR, IGLCR and IGLL5, the method can be used to additionally discriminate false negatives, therefore providing even more accurate assays than traditional methods for detecting B cell clonality. The pattern in individual B cells of only signal associated with hybridized IGKCR probe indicates kappa restriction in the sample. As discussed above, this pattern was known but can be determined at the level of a single nucleic acid molecule and at the spatial resolution of an individual cell using methods of the invention. The pattern in individual B cells of only signal associated with hybridized IGLCR probe indicates lambda restriction in the sample. Similarly, this pattern was known but can be determined at the level of a single nucleic acid molecule and at the spatial resolution of an individual cell using methods of the invention. The pattern of detecting both signal associated with hybridized IGKCR probe and signal associated with hybridized IGLCR probe in separate B cells indicates the sample is polyclonal. Again, this pattern was known but can be determined at at the level of a single nucleic acid molecule and at the spatial resolution of an individual cell using methods of the invention.

The pattern of both signal associated with hybridized IGKCR probe and signal associated with hybridized IGLCR probe in the same B cell indicates the sample is monoclonal. As discussed above, the observation of this signal pattern allows the discrimination of a clonal, and therefore neoplastic B cell population, that with previous methods would likely have been identified as polyclonal and therefore negative for neoplastic cells. Thus, the ability of the methods of the invention to discriminate this pattern of detection of IGKCR and IGLCR hybridized probes within the same cell results in a reduction in false negative determinations and a higher accuracy assay.

As described herein, the high sensitivity of the methods of the invention led to further unexpected observations regarding patterns of signal associated with IGKCR, IGLCR and IGLL5 probes. Such observations provide for further reduction in false negatives. According to the invention, the pattern where both signal associated with IGKCR probe and signal associated with IGLCR probe are present in the same B cell, and where the level of signal associated with hybridized IGLL5 probe is greater than or equal to the level of signal associated with hybridized IGLCR probe, indicates kappa restriction. This observation is based on the recognition that, in some situations, an apparent signal associated with lambda probes actually relate to cross-hybridization with IGLL5. Since IGLL5 occurs in non-rearranged lambda gene, the cells are in fact kappa restricted and clonal. Again, the high sensitivity of the assay methods of the invention provides a reduction in false negatives.

According to the invention, the pattern where both signal associated with IGKCR probe and signal associated with IGLCR probe are present in the same B cell, and where the level of signal associated with hybridized IGLL5 probe is less than the level of signal associated with hybridized IGLCR probe, indicates a clonal, non-restricted sample. This observation was also surprising and obtainable based on the high sensitivity of assays of the invention (see Example). As discussed herein, immunoglobulin light chain restriction is used as a hallmark for B cell clonality and determination of neoplasms. The observation that a non-restricted B cell was in fact clonal was surprising and again provides a situation where a sample likely to have been identified as a negative due to it being non-restricted is in fact a clonal, non-restricted sample.

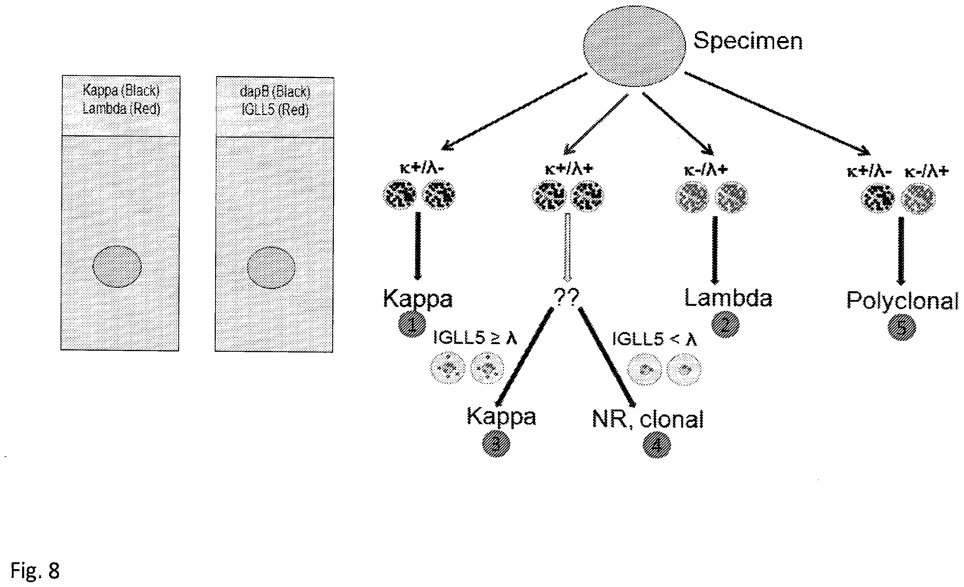

The methods of the invention have allowed the development of an algorithm for determining light chain restriction and clonality at a higher level of accuracy (see Example and Figure 8). Briefly, a specimen is analyzed by methods of the invention, which provide detection sensitivity down to the single molecule level and spatial resolution down to the individual cell level. This high sensitivity detection, which is significantly more sensitive than previous methods, has provided insights into previously unrecognized stratification of B cell populations that are clonal but would have otherwise been characterized by previous assay methods as being polyclonal and therefore benign. The methods of the invention can be used to visualize individual cells and even individual nucleic acid molecules within the cells. In the case of traditional B cell analysis, the presence of kappa restricted clonal cells (Figure 8, pattern 1), lambda restricted clonal cells (Figure 8, pattern 2), and polyclonal cells that individually express either kappa or lambda but as a population have both kappa and lambda expression (Figure 8, pattern 5) have been used to determine immunoglobulin restriction and clonality of B cells.

Due to the high level of sensitivity of the methods of the invention, additional patterns not previously observed were identified. These additional patterns relate to clonal populations that exhibit both kappa and lambda probe signals, which would have likely been determined to be benign, polyclonal populations using previous assay methods. At the individual cell level, both kappa and lambda signals were observed within the same cell (Figure 8, patterns 3 and 4). At a lower resolution of previous assays, the presence of both kappa and lambda signals would likely be characterized as being a benign, polyclonal cell population. The methods of the invention provide the ability to discriminate these clonal populations from polyclonal populations. In one pattern (pattern 3), the presence of kappa and lambda signal was found to be caused by cross-hybridization with immunoglobulin like polypeptide 5 (IGLL5). This protein is expressed from unrearranged lambda DNA. Therefore, although the signal corresponds to hybridized lambda probes, the cells do not express rearranged lambda, but rather are actually kappa restricted. This can be distinguished by determining the relative expression of IGLL5 and lambda. In pattern 3, IGLL5 is expressed at greater or equal to levels as lambda expression, and the cell population is therefore kappa restricted and clonal.

In addition, a further pattern of expression was discovered using the methods of the invention. In this additional pattern (pattern 4), both kappa and lambda are expressed. This pattern can be distinguished from pattern 3 by similarly determining the relative expression of IGLL5 and lambda. In pattern 4, expression of IGLL5 at lower levels than lambda indicates that the signal associated with lambda probe corresponds to lambda expression. This pattern of expression corresponds to a non-restricted but clonal population of B cells. Thus, the methods of the invention provide highly sensitive assays that can be used to further stratify B cell populations and accurately identify clonal B cell populations that would be characterized as benign, polyclonal populations using previously known assays. The methods of the invention therefore are particularly useful as highly accurate and highly sensitive assays for detecting immunoglobulin restriction and B cell clonality and provide significant reduction in false negatives.

The results disclosed herein using the methods of the invention provide for a highly sensitive and more accurate assessment of B cell neoplasms. The methods of the invention are able to discriminate and identify clonal B cells that are neoplastic that would have been likely categorized as negative, polynclonal cells. Thus, the methods of the invention provide superior results over previous assays for detecting immunoglobulin light chain restriction and clonality in B cells. The methods of the invention can therefore be used in the diagnosis of B cell neoplasms. The invention therefore provides an embodiment of diagnosing a B cell neoplasm using methods of the invention, as disclosed herein. The methods of the invention are particularly useful for assessing neoplasms in B cell types having low expression of light chains and/or the unexpected observations described herein of signals associated with kappa and lambda expression, both of which would have resulted in a false negative or indiscriminate outcome using previous methods.

The methods of the invention are also useful for predicting and/or monitoring therapeutic outcomes of treatment for a B cell neoplastic condition. Based on the high sensitivity of the methods of the invention, the methods can be used to monitor disease progression and/or remission. This can be particularly useful since the methods of the invention are more sensitive than previous methods and can detect kappa and lambda signals at the level of a single nucleic acid molecule and at the spatial resolution of an individual cell. Because the methods of the invention are more sensitive, they can be particularly useful for monitoring disease regression in response to treatment since the number of neoplastic B cells would be expected to be lower. Thus, in a further embodiment, the invention provides a method of monitoring responsiveness to therapy using methods of the invention.

As disclosed herein, one method for performing *in situ* hybridization based assays of the invention include the use of an RNAscope® assay. RNAscope® *in situ* hybridization assays are commercially available from Advanced Cell Diagnostics, Inc. (Hayward CA). RNAscope® assays have been described previously, for example, in U.S. Patent No. 7,709,198, U.S. publications 2008/0038725 and 2009/0081688, and WO 2007/001986 and WO 2007/002006. RNAscope® assays in various embodiments are described below in more detail. It is understood that the assays of the invention can be performed with commercially available RNAscope® *in situ* hybridization assays or variations of such assays as described herein.

As used herein, the term "label probe" refers to an entity that binds to a target molecule, directly or indirectly, and allows the target to be detected. A label probe (or "LP") contains a nucleic acid binding portion that is typically a single-stranded polynucleotide or oligonucleotide that comprises one or more labels which directly or indirectly provides a detectable signal. The label can be covalently attached to the polynucleotide, or the polynucleotide can be configured to bind to the label. For example, a biotinylated polynucleotide can bind a streptavidin-associated label. The label probe can, for example, hybridize directly to a target nucleic acid, such as IGKCR, IGLCR or IGLL5, or it can hybridize to a nucleic acid that is in turn hybridized to the target nucleic acid or to one or more other nucleic acids that are hybridized to the target nucleic acid. Thus, the label probe can comprise a polynucleotide sequence that is complementary to a polynucleotide sequence, particularly a portion, of the target nucleic acid. Alternatively, the label probe can comprise at least one polynucleotide sequence that is complementary to a polynucleotide sequence in an amplifier, preamplifier, intermediary complex, or the like, as described herein. As used herein, a label probe comprising an enzyme label refers to a label probe comprising a nucleic acid binding portion such as an oligonucleotide and an enzyme or non-enzyme label that is coupled to the nucleic acid binding portion. As disclosed herein, the coupling of the enzyme or non-enzyme label to the nucleic acid binding portion can be covalent or through a high affinity binding interaction such as biotin/avidin or other similar high affinity binding molecules.

As used herein, a "target probe" is a polynucleotide that is capable of hybridizing to a target nucleic acid and capturing or binding a label probe or intermediary complex molecule to that target nucleic acid. The target probe can hybridize directly to the label probe, or it can hybridize to one or more nucleic acids that in turn hybridize to the label probe; for example, the target probe can hybridize to an amplifier or a preamplifier in an intermediary complex. The target probe thus includes a first polynucleotide sequence that is complementary to a polynucleotide sequence of the target nucleic acid and a second polynucleotide sequence that is complementary to a polynucleotide sequence of the label probe, amplifier, preamplifier, or the like. The target probe is generally single-stranded so that the complementary sequence is available to hybridize with a corresponding target nucleic acid, label probe, amplifier or preamplifier.

As used herein, an "amplifier" is a molecule, typically a polynucleotide, that is capable of hybridizing to multiple label probes. Typically, the amplifier hybridizes to multiple identical label probes. The amplifier can also hybridize to a target nucleic acid, at least one target probe or nucleic acid bound to a target probe such as a preamplifier. For example, the amplifier can hybridize to at least one target probe and to a plurality of label probes, or to a preamplifier and a plurality of label probes. The amplifier can be, for example, a linear, forked, comb-like, or branched nucleic acid. As described herein for all polynucleotides, the amplifier can include modified nucleotides and/or nonstandard internucleotide linkages as well as standard deoxyribonucleotides, ribonucleotides, and/or phosphodiester bonds. Suitable amplifiers are described, for example, in U.S. Patent Nos. 5,635,352, 5,124,246, 5,710,264, 5,849,481, and 7,709,198 and U.S. publications 2008/0038725 and 2009/0081688.

As used herein, a "preamplifier" is a molecule, typically a polynucleotide, that serves as an intermediate between one or more target probes and one or more amplifiers. Typically, the preamplifier hybridizes simultaneously to one or more target probes and to a plurality of amplifiers. Exemplary preamplifiers are described, for example, in U.S. Patent Nos. 5,635,352, 5,681,697 and 7,709,198 and U.S. publications 2008/0038725 and 2009/0081688.

As used herein, an "intermediary complex" is a molecule, and can be a large, complex molecule, or an assembly of multiple molecules, that has one or more components, each containing a section capable of binding specifically to a section of the target nucleic acid and has another component or multiple components each containing one or multiple sections capable of binding to the label probe.

In one embodiment, a method of detecting two or more target nucleic acids in a sample, such as IGKCR, IGLCR, or IGLL5, can be carried out by (a) contacting a sample with two or more label probes, wherein each label probe comprises a distinct enzyme label or non-enzyme label and targets a distinct nucleic acid target; (b) attaching or binding the two or more label probes to a first target nucleic acid and a second target nucleic acid in the sample by hybridization; (c) contacting the sample with a first substrate for the first enzyme of the first label probe; (d) reacting the first substrate with the first enzyme, thereby producing a first detectable signal associated with the first target nucleic acid molecule; (e) contacting the sample with a second substrate for the second enzyme of the second label probe; (f) reacting the second substrate with the second enzyme, thereby producing a second detectable signal associated with the second target nucleic acid molecule; and (g) detecting the first detectable signal and the second detectable signal, thereby detecting the first and second target nucleic acids in the sample.

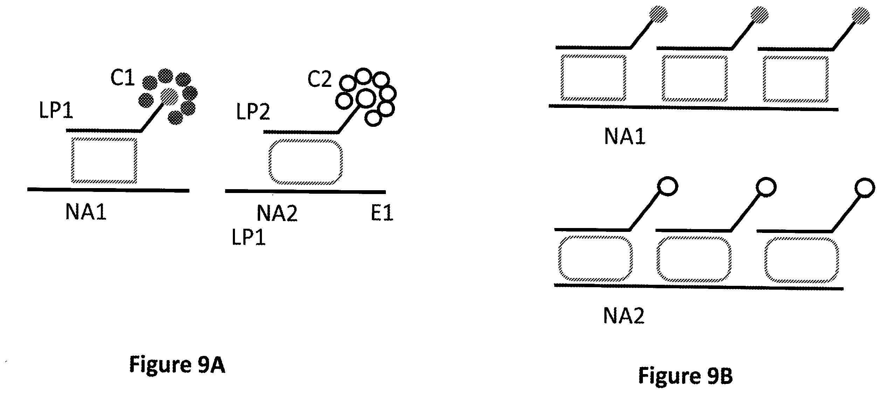

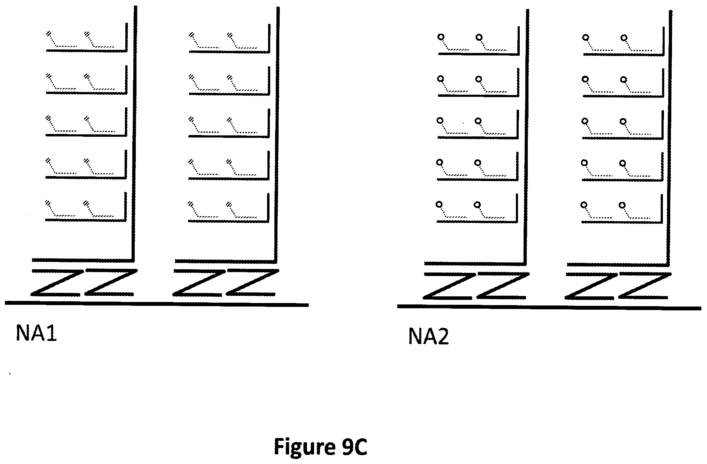

Exemplary embodiments of probe configurations are shown in Figure 9. In more detail, Figure 9A shows an exemplary embodiment, where the detection of two nucleic acid targets (NA1 and NA2) is shown. It is understood by those skilled in the art that such nucleic acid targets are distinct from each other in that they are non-identical nucleic acid molecules having different nucleic acid sequences. For example, the target nucleic acid can be IGKCR, IGLCR or ILL5C mRNA. Such distinct nucleic acid molecules can be distinguished from each other by selection of appropriate regions of the nucleic acid target such that specific and distinguishable binding to the nucleic acid targets can be achieved. As described herein, in some cases additional steps can be utilized to distinguish two different nucleic acid targets if there is substantial overlap and homology between two targets (see Example and relationship between IGLCR and IGLL5). Such a selection of appropriate regions and design of specific and selective reagents that bind to the target nucleic acids, in particular oligonucleotides or probes that specifically and selectively bind to a target nucleic acid, are well known to those skilled in the art (see Sambrook et al., Molecular Cloning: A Laboratory Manual, Third Ed., Cold Spring Harbor Laboratory, New York (2001); Ausubel et al., Current Protocols in Molecular Biology, John Wiley and Sons, Baltimore, MD (1999). A desired specificity can be achieved using appropriate selection of regions of a target nucleic acid as well as appropriate lengths of a binding agent such as an oligonucleotide or probe, and such selection methods are well known to those skilled in the art. Thus, one skilled in the art will readily understand and can readily determine appropriate reagents, such as oligonucleotides or probes, that can be used to target one particular target nucleic acid over another target nucleic acid.

As depicted in Figure 9A, a sample containing the distinct target nucleic acids (NA1 and NA2) can be contacted with two label probes. Each of the label probes is specific for a target nucleic acid. The embodiment depicted in Figure 9A shows the label probe being bound to the target nucleic acid by way of an intermediary complex (discussed below). However, one skilled in the art can readily envision using a label probe that directly binds to the target nucleic acid, if desired. The specificity of a label probe for a target nucleic acid is achieved by (1) selection of a suitable nucleic acid target binding region that is an oligonucleotide complementary to the target nucleic acid, and (2) coupling the binding region comprising the oligonucleotide to a specific enzyme. Alternatively, other types of labels, as disclosed herein, can be used, for example, a fluorescent or chromogenic label using chromophores or fluorophores. Accordingly, the description below describing the use of enzymes as the label can be similarly carried out using other detectable labels, as disclosed herein. The label probe thereby provides the association of a detectable agent, in this case an enzyme, with a particular nucleic acid target. In the case of an enzyme label, the enzymatic activity of the enzyme is utilized to generate a detectable signal. As shown in Figure 9A, the label probe that targets a first nucleic acid target is distinct from a label probe that targets a second nucleic acid target. The label probe for the first nucleic acid target (LP1) comprises a binding region, that is, an oligonucleotide that is complementary to and can specifically and selectively hybridize with nucleic acid 1, and a first specific label such as an enzyme, whereas the second label probe (LP2) comprises a binding region, that is, an oligonucleotide that is complementary to and can specifically and selectively hybridize with nucleic acid 2 and a second specific label such as an enzyme, where the first and second specific labels, for example, enzymes or non-enzyme labels, are distinct from each other. Exemplary specific and distinct enzymes can be selected from horseradish peroxidase, alkaline phosphatase, β-galactosidase and glucose oxidase, and the like, as disclosed herein. Thus, the label probe is designed such that it can specifically and selectively target a particular target nucleic acid and associate a distinguishable label to the target nucleic acid by utilizing distinct labels such as enzymes or non-enzyme labels on label probes for different target nucleic acids.

As described herein, the methods of the invention utilize distinct enzymes or non-enzyme labels that allow the association of a distinguishable detectable label with a target nucleic acid. Any of a number of enzymes or non-enzyme labels can be utilized so long as the enzymatic activity or non-enzyme label, respectively, can be detected. The enzyme thereby produces a detectable signal, which can be utilized to detect a target nucleic acid. Particularly useful detectable signals are chromogenic or fluorogenic signals. Accordingly, particularly useful enzymes for use as a label include those for which a chromogenic or fluorogenic substrate is available. Such chromogenic or fluorgenic substrates can be converted by enzymatic reaction to a readily detectable chromogenic or fluorescent product, which can be readily detected and/or quantified using microscopy or spectroscopy. Such enzymes are well known to those skilled in the art, including but not limited to, horseradish peroxidase, alkaline phosphatase, β-galactosidase, glucose oxidase, and the like (see Hermanson, Bioconjugate Techniques, Academic Press, San Diego (1996)). Other enzymes that have well known chromogenic or fluoregenic substrates include various peptidases, where chromogenic or fluorogenic peptide substrates can be utilized to detect proteolytic cleavage reactions. The use of chromogenic and fluorogenic substrates is also well known in bacterial diagnostics, including but not limited to the use of α- and β-galactosidase, β-glucuronidase,6-phospho-β-D-galatoside 6-phosphogalactohydrolase, β-gluosidase, α-glucosidase, amylase, neuraminidase, esterases, lipases, and the like (Manafi et al., Microbiol. Rev. 55:335-348 (1991)), and such enzymes with known chromogenic or fluorogenic substrates can readily be adapted for use in methods of the present invention.

Various chromogenic or fluorogenic substrates to produce detectable signals are well known to those skilled in the art and are commercially available. Exemplary substrates that can be utilized to produce a detectable signal include, but are not limited to, 3,3'-diaminobenzidine (DAB), 3,3',5,5'-tetramethylbenzidine (TMB), Chloronaphthol (4-CN)(4-chloro-1-naphthol), 2,2'-azino-bis(3-ethylbenzothiazoline-6-sulphonic acid) (ABTS), o-phenylenediamine dihydrochloride (OPD), and 3-amino-9-ethylcarbazole (AEC) for horseradish peroxidase; 5-bromo-4-chloro-3-indolyl-1-phosphate (BCIP), nitroblue tetrazolium (NBT), Fast Red (Fast Red TR/AS-MX), and p-Nitrophenyl Phosphate (PNPP) for alkaline phosphatase; 1-Methyl-3-indolyl-β-D-galactopyranoside and 2-Methoxy-4-(2-nitrovinyl)phenyl β-D-galactopyranoside for β-galactosidase; 2-Methoxy-4-(2-nitrovinyl)phenyl β-D-glucopyranoside for β-glucosidase; and the like. Exemplary fluorogenic substrates include, but are not limited to, 4-(Trifluoromethyl)umbelliferyl phosphate for alkaline phosphatase; 4-Methylumbelliferyl phosphate bis (2-amino-2-methyl-1,3-propanediol), 4-Methylumbelliferyl phosphate bis (cyclohexylammonium) and 4-Methylumbelliferyl phosphate for phosphatases; QuantaBlu™ and QuantaRed™ for horseradish peroxidase; 4-Methylumbelliferyl β-D-galactopyranoside, Fluorescein di(β-D-galactopyranoside) and Naphthofluorescein di-(β-D-galactopyranoside) for β-galactosidase; 3-Acetylumbelliferyl β-D-glucopyranoside and 4-Methylumbelliferyl-β- D-glucopyranoside for β-glucosidase; and 4-Methylumbelliferyl-α- D-galactopyranoside for α-galactosidase. Exemplary enzymes and substrates for producing a detectable signal are also described, for example, in US publication 2012/0100540. Various detectable enzyme substrates, including chromogenic or fluorogenic substrates, are well known and commercially available (Pierce, Rockford IL; Santa Cruz Biotechnology, Dallas TX; Invitrogen, Carlsbad CA; 42 Life Science; Biocare). Generally, the substrates are converted to products that form precipitates that are deposited at the site of the target nucleic acid. Other exemplary substrates include, but are not limited to, HRP-Green (42 Life Science), Betazoid DAB, Cardassian DAB, Romulin AEC, Bajoran Purple, Vina Green, Deep Space Black™, Warp Red™, Vulcan Fast Red and Ferangi Blue from Biocare (Concord CA; biocare.net/products/detection/chromogens).

Biotin-avidin (or biotin-streptavidin) is a well known signal amplification system based on the facts that the two molecules have extraordinarily high affinity to each other and that one avidin/streptavidin molecule can bind four biotin molecules. Antibodies are widely used for signal amplification in immunohistochemistry and ISH. Tyramide signal amplification (TSA) is based on the deposition of a large number of haptenized tyramide molecules by peroxidase activity. Tyramine is a phenolic compound. In the presence of small amounts of hydrogen peroxide, immobilized Horse Radish Peroxidase (HRP) converts the labeled substrate into a short-lived, extremely reactive intermediate. The activated substrate molecules then very rapidly react with and covalently bind to electron-rich moieties of proteins, such as tyrosine, at or near the site of the peroxidase binding site. In this way, a lot of extra hapten molecules conjugated to tyramide can be introduced at the hybridization site *in situ.* Subsequently, the deposited tyramide-hapten molecules can be visualized directly or indirectly. Such a detection system is described in more detail in U.S. publication 2012/0100540.

Embodiments described herein can utilize enzymes to generate a detectable signal using appropriate chromogenic or fluorogenic substrates. It is understood that, alternatively, a label probe can have a detectable label directly coupled to the nucleic acid portion of the label probe. Exemplary detectable labels are well known to those skilled in the art, including but not limited to chromogenic or fluorescent labels (see Hermanson, Bioconjugate Techniques, Academic Press, San Diego (1996)). Exemplary fluorophores useful as labels include, but are not limited to, rhodamine derivatives, for example, tetramethylrhodamine, rhodamine B, rhodamine 6G, sulforhodamine B, Texas Red (sulforhodamine 101), rhodamine 110, and derivatives thereof such as tetramethylrhodamine-5-(or 6), lissamine rhodamine B, and the like; 7-nitrobenz-2-oxa-1,3-diazole (NBD); fluorescein and derivatives thereof; napthalenes such as dansyl (5-dimethylaminonapthalene-1-sulfonyl); coumarin derivatives such as 7-amino-4-methylcoumarin-3-acetic acid (AMCA), 7-diethylamino-3-[(4'-(iodoacetyl)amino)phenyl]-4-methylcoumarin (DCIA), Alexa fluor dyes (Molecular Probes), and the like; 4,4-difluoro-4-bora-3a,4a-diaza-s-indacene (BODIPY™) and derivatives thereof (Molecular Probes; Eugene Oreg.); pyrenes and sulfonated pyrenes such as Cascade Blue™ and derivatives thereof, including 8-methoxypyrene-1,3,6-trisulfonic acid, and the like; pyridyloxazole derivatives and dapoxyl derivatives (Molecular Probes); Lucifer Yellow (3,6-disulfonate-4-amino-naphthalimide) and derivatives thereof; CyDye™ fluorescent dyes (Amersham/GE Healthcare Life Sciences; Piscataway NJ), and the like. Exemplary chromophores include, but are not limited to, phenolphthalein, malachite green, nitroaromatics such as nitrophenyl, diazo dyes, dabsyl (4-dimethylaminoazobenzene-4'-sulfonyl), and the like

In one embodiment, label probes are directly hybridized to the target nucleic acid molecules. As discussed above, in such an embodiment the label probe itself comprises a nucleic acid target binding region, such as a complementary oligonucleotide, and binds directly to the target nucleic acid. Alternatively, the label probe can be bound to the target nucleic acid indirectly using an intermediary complex. Such an embodiment is depicted in Figure 9A. In one such embodiment, the intermediary complex can be a single molecule such as a single nucleic acid molecule. For example, an intermediary complex that is a single molecule can comprise a region that is capable of specifically and selectively attaching or binding to a target nucleic acid. This is similar to a label probe that is designed to attach or bind to a target nucleic acid, except that the binding to the nucleic acid target is mediated by the intermediary molecule. The intermediary molecule also can comprise a region that is capable of specifically and selectively binding to a label probe (see Figure 9A). In such a case, the label probe and intermediary molecule are designed to have binding regions such as oligonucleotide regions that are complementary to each other. Such a configuration thereby associates the enzyme label to a target nucleic acid specifically and selectively through an intermediary molecule. As further depicted in Figure 9A, a second target nucleic acid can be specifically and selectively associated with a distinct enzyme or non-enzyme label through a second intermediary complex. The second intermediary complex comprises a first region that can specifically and selectively bind to the target nucleic acid and a second region that can specifically and selectively bind to the second label probe. Thus, the label probes can be bound to the target nucleic acid molecules by a first and second intermediary complex (see Figure 9A). Further, the intermediary complex can comprise a molecule comprising a first region complementary to the target nucleic acid and the same or a different molecule comprising at least one second region complementary to the label probe. Further still, the intermediary complex can comprise an assembly of multiple molecules, of which one or more components each comprising one or more regions complementary to the target nucleic acid and one or more other components each comprising one or more regions complementary to the label probe. In this way, the intermediary complex can be an amplifying structure allowing multiple label probes to be attached to a region on a target nucleic acid. In general, the intermediary complex comprises one or more nucleic acid molecules to take advantage of the specific and selective binding capabilities of nucleic acid interactions, as described herein.

As described herein, a sample can be contacted with two or more label probes, where each label probe comprises a distinct enzyme or non-enzyme label and targets a distinct nucleic acid target. The label probes are bound to the target nucleic acid molecules by hybridization of complementary regions on the target nucleic acid and either the label probe or the intermediary complex, as discussed above. In particular, the two or more label probes are contacted with the sample simultaneously so that the label probes are bound to the respective target nucleic acids in the same reaction. This is particularly useful in that association of label with each of the target nucleic acids can occur at the same time, thereby reducing assay time and reagent costs. This contrasts with other methods, where hybridization reactions for each target nucleic acid are carried out sequentially. Nevertheless, it is understood that methods of the invention can be preformed with sequential hybridization steps, if desired.

As described herein, the label probe used in methods of the invention can comprise a target nucleic acid binding region comprising an oligonucleotide, which is complementary to the target nucleic acid or complementary to a component of an intermediary complex that can bind to the target nucleic acid. In such a configuration of a label probe, the oligonucleotide portion is coupled to an enzyme or non-enzyme label. This differs from previous methods, where an enzyme is used to detect a nucleic acid target through an antibody, where the antibody is detected using well known immunodetection methods. The label probe configuration used in methods of the present invention are particularly useful because the enzyme label or non-enzyme label is coupled to an oligonucleotide that specifically and selectively binds to a target nucleic acid. The coupling of the enzyme or non-enzyme label to the oligonucleotide in the label probe can be covalent, using well known chemical coupling methods (see, for example, Hermanson, Bioconjugate Techniques, Academic Press, San Diego (1996)). Alternatively, a high affinity interaction such as biotin/avidin, where biotin or avidin is coupled to the oligonucleotide and enzyme or non-enzyme label in the label probe, can be used to couple the enzyme or non-enzyme label to the oligonucleotide in the label probe (see US publication 2012/0100540). It is understood that the coupling of the enzyme or non-enzyme label to the oligonucleotide in the label probe is of sufficient affinity, such as through an interaction such as biotin/avidin, or is covalent such that the enzyme or non-enzyme label remains attached to the oligonucleotide throughout various reactions and assay conditions of the methods of the invention.

Once the label probes are bound to the target nucleic acids, either directly or via an intermediary complex, the sample is contacted with a first substrate for the first enzyme of the first label probe when an enzyme is used as the label. Such a reaction is depicted in Figure 9A. By using a first distinct enzyme and an appropriate substrate, a detectable signal can be generated that is associated with the target nucleic acid and is therefore distinct for the first target nucleic acid. Alternatively, a distinct detectable label can be used that is not an enzyme and does not require the use of an enzyme substrate to generate detectable signal. Since the reaction conditions for a distinct enzyme are often different, and utilize a different substrate, the methods of the invention can be carried out such that a second substrate for the second distinct enzyme is added sequentially, after the reaction with the first enzyme has taken place. Optionally, the first enzyme can be inactivated prior to sequential addition of the second substrate for the second distinct enzyme. It is understood that, if the distinct enzymes are compatible, the first and second enzymatic reactions can be carried out concurrently. In a particular embodiment, the first enzymatic reaction is carried out and the detectable signal generated, followed sequentially by the second enzymatic reaction and production of the second detectable signal (see Figure 9A). The sequential steps can be carried out optionally with an intervening wash step, in particular if the reaction for the second enzyme is not compatible with reaction conditions for the first enzyme.

As described herein, distinct enzymes or non-enzyme labels are associated with a target nucleic acid using label probes that target distinct nucleic acid targets. The association of a distinct enzyme with a target nucleic acid allows the use of the enzymatic activity of the enzyme to generate a detectable signal associated with a target nucleic acid. In particular, a chromogenic or fluorogenic substrate can be used to produce a detectable chromogenic or fluorescent signal associated with a target nucleic acid. Since distinct enzymes are associated with distinct target nucleic acids, the detectable signals are associated with the respective target nucleic acids, thereby allowing detection of the target nucleic acids. Alternatively, distinct non-enzyme labels can be used for distinctly labeling the two or more target nucleic acids. As described herein, well known methods such as microscopy or spectroscopy can be utilized to visualize chromogenic or fluorescent detectable signals associated with the respective target nucleic acids. In general, either chromogenic substrates or fluorogenic substrates, or chromogenic or fluorescent labels, will be utilized for a particular assay so that a single type of instrument can be used for detection of nucleic acid targets in the same sample.

In another embodiment, a method of detecting two or more target nucleic acids can be performed by (a) contacting a sample with two or more sets of label probes, wherein each set of label probes comprises a plurality of label probes, wherein the plurality of label probes within a set have the same enzyme label or non-enzyme label, and optionally wherein the plurality of label probes comprise label probes that bind to different regions of a target nucleic acid molecule; and wherein each set of label probes comprises a distinct enzyme or non-enzyme label and targets a distinct nucleic acid target; (b) attaching or binding the two or more sets of label probes to a first target nucleic acid and a second target nucleic acid in the sample by hybridization; (c) contacting the sample with a first substrate for the first enzyme of the first set of label probes if an enzyme label is used; (d) reacting the first substrate with the first enzyme, thereby producing a first detectable signal associated with the first target nucleic acid molecule; (e) contacting the sample with a second substrate for the second enzyme of the second set of label probes if an enzyme label is used; (f) reacting the second substrate with the second enzyme, thereby producing a second detectable signal associated with the second target nucleic acid molecule; and (g) detecting the first detectable signal and the second detectable signal, thereby detecting the first and second target nucleic acids in the sample.

The methods can include the step of contacting a sample with two or more sets of label probes, wherein each set of label probes comprises a plurality of label probes, wherein the plurality of label probes within a set have the same enzyme or non-enzyme label, and optionally wherein the plurality of label probes comprise label probes that attach or bind to different regions of a target nucleic acid molecule; and wherein each set of label probes comprises a distinct enzyme or non-enzyme label and targets a distinct nucleic acid target; attaching or binding the two or more sets of label probes to two or more target nucleic acids, for example, at least a first target nucleic acid and a second target nucleic acid, or additional target nucleic acids, in the sample by hybridization; contacting the sample with a first substrate for the first enzyme of the first set of label probes if an enzyme is the label; reacting the first substrate with the first enzyme, thereby producing a first detectable signal associated with the first target nucleic acid molecule; contacting the sample with a second substrate for the second enzyme of the second set of label probes if an enzyme is the label; reacting the second substrate with the second enzyme, thereby producing a second detectable signal associated with the second target nucleic acid molecule; and detecting the first detectable signal and the second detectable signal, thereby detecting the first and second target nucleic acids in the sample. Alternatively, distinct detectable labels directly coupled to the label probe can be used.

An embodiment of the invention utilizing sets of probes is depicted in Figure 9B. As shown in Figure 9B, a set of label probes is employed, where label probes within the set target different regions of the target nucleic acid. One skilled in the art will readily understand that, even though the embodiment depicted in Figure 9B shows binding of label probe to the target nucleic acid via an intermediary complex, a configuration can be used where the label probe directly binds to the target nucleic acid. In general, a set of label probes for a target nucleic acid will hybridize to non-overlapping regions of the target nucleic acid. This allows each of the label probes within the set to bind independently of each other and non-competitively, thereby maximizing signal. Although the label probes within the set bind to different regions of the target nucleic acid, the same distinct enzyme, or distinct non-enzyme label, is coupled to each of the label probes within the set. Thus, instead of having a single region of the target nucleic acid being bound by the label probe, as depicted in Figure 9A, multiple label probes are bound to the target nucleic acid. Since the same distinct enzyme, or distinct non-enzyme label, is coupled to each of the label probes in the set, multiple copies of the enzyme are bound to the target nucleic acid. This configuration can therefore be used to substantially increase the detectable signal associated with the target nucleic acid.

As described above, the label probes in a set of label probes can be directly hybridized to the target nucleic acid molecules. Alternatively, the label probes can be bound to the target nucleic acid molecules by a first and second intermediary complex (see Figure 9B). As described herein, the intermediary complex can comprise a molecule comprising a first region complementary to the target nucleic acid and the same or another molecule comprising at least one second region complementary to the label probe (see Figure 9B). As depicted in Figure 9B, using sets of label probes provides for increased detectable signal to be associated with the respective target nucleic acids.

In still another embodiment, the methods of the invention can employ an intermediary complex, where the intermediary complex comprises multiple molecules rather than a single molecule. Such an intermediary complex is particularly useful for amplifying the detectable signal, providing higher sensitivity detection of target nucleic acids. Such methods for amplifying a signal are described, for example, in U.S. Patent Nos. 5,635,352, 5,124,246, 5,710,264, 5,849,481, and 7,709,198, and U.S. publications 2008/0038725 and 2009/0081688, as well as WO 2007/001986 and WO 2012/054795.

In one embodiment utilizing an intermediary complex, the intermediary complex can comprise an amplifier or preamplifier. Such an embodiment is depicted in Figure 9C. In particular, Figure 9C shows an intermediary complex, where an amplifier is used. With the use of an amplifier, which itself comprises a target nucleic acid binding region, multiple copies of the label probe can be attached to the target nucleic acid with a single amplifier. Although depicted in Figure 9C as two label probes being bound to the amplifier, it is understood that this is merely exemplary and that multiple copies of the label probe can be bound to the amplifier, as desired. Thus, a plurality of label probes can be hybridized to one or more amplifiers. Similar to the embodiment shown in Figure 9B, multiple amplifiers can be used that bind to different regions of the target nucleic acid, thereby providing even greater signal amplification with each amplifier being bound to multiple copies of the label probe. As with other embodiments described herein, the label probes for targeting a particular nucleic acid target are coupled to the same enzyme or same distinct non-enzyme label. It is understood that, even though Figure 9C depicts the use of a preamplifier, a probe configuration can be used where an amplifier is designed to directly bind to a target nucleic acid without the use of a preamplifier.

In another embodiment depicted in Figure 9C, the intermediary complex can comprise an amplifier and preamplifier. In this configuration, the preamplifier comprises a nucleic acid target binding region and multiple binding sites for amplifiers. Thus, a plurality of amplifiers can be hybridized to a preamplifier. Again the amplifiers can contain multiple binding sites for the label probe, and the label probes used for a particular nucleic acid target comprise the same enzyme or non-enzyme label so that the same distinct enzyme label, or distinct non-enzyme label, is associated with the target nucleic acid. In a configuration using a preamplifier, the preamplifier is hybridized to the target nucleic acid. Although depicted in Figure 9C with a target probe, as described below, it is understood that a preamplifier can be designed so that it directly binds to the target nucleic acid, if desired.

In still another embodiment, the intermediary complex can comprise a target probe, amplifier and preamplifier. Such an embodiment is depicted in Figure 9C. Similar to the embodiment described above, a preamplifier is used with multiple amplifiers bound, and multiple label probes are bound to the amplifiers. However, instead of the preamplifier binding directly to the nucleic acid target, a target probe is used. By using a target probe, the preamplifier is not required to have a nucleic acid target binding region. Instead, the target probe is designed to comprise a binding region for the nucleic acid target. If more than one target probe is used, multiple target probes can be designed to bind to different regions of the target nucleic acid, as shown in Figure 9C. In such a configuration, the same preamplifier can be used for multiple binding events through the target probe instead of designing and generating different preamplifiers for targeting different regions of the target nucleic acid. Although depicted in Figure 9C as a "Z" configuration, it is understood that any suitable configuration can be used so long as the target probe comprises regions complementary to a target nucleic acid and a preamplifier. Further, it is understood that a single target probe can used to bind the preamplifier rather than two target probes for each amplifier as depicted in Figure 9C.

Thus, in a particular embodiment the intermediary complex can comprise a target probe, amplifier and preamplifier (see Figure 9C). In addition, a plurality of label probes can be hybridized to one or more amplifiers, a plurality of amplifiers can be hybridized to a preamplifier, and the preamplifier can be hybridized to a target probe.

In yet another embodiment, a configuration similar to that depicted in Figure 9C can be used, where the intermediate complex comprises a target probe, preamplifier and amplifier. Rather than using a single target probe to bind the preamplifier to the target nucleic acid, two or more target probes are used to bind the preamplifier to the target nucleic acid (see Figure 9C). In general, the advantages of such a configuration can be realized using two target probes, that is, a pair of target probes to bind a preamplifier to the target nucleic acid. Such a configuration and its advantages for increasing sensitivity and decreasing background are described, for example, in U.S. Patent No. 7,709,198, U.S. publications 2008/0038725 and 2009/0081688, and WO 2007/001986 and WO 2007/002006. U.S. Patent No. 7,709,198 and U.S. publications 2008/0038725 and 2009/0081688 additionally describe details for selecting characteristics of the target probes, including target probe pairs, including length, orientation, hybridization conditions, and the like, as discussed below in more detail.

As disclosed herein, even though the figures generally depict detection of one or two target nucleic acids, it understood that such methods can be readily applied to one target nucleic acid or to multiplex detection of two or more nucleic acid targets, for example, 3, 4, 5, 6, 7, 8, 9, 10, or even larger numbers of distinct nucleic acid targets. For example, an assay to detect IGKCR, IGLCR and/or IGLL5 mRNA can be performed in methods of the invention. All that is required is the availability of a suitable number of distinct enzymes or non-enzyme labels for use as a distinct label for each of the target nucleic acids. Thus, the methods of the invention can be applied to detect multiple nucleic acid targets, in particular two or more nucleic acid targets, in a sample.

It is understood by those skilled in the art that any of a number of suitable samples can be used for detecting target nucleic acids using methods of the invention. The sample for use in methods of the invention will generally be a biological sample or tissue sample. Such a sample can be obtained from a biological subject, including a sample of biological tissue or fluid origin that is collected from an individual or some other source of biological material such as a biopsy. A biological sample also includes samples from a region of a biological subject containing or suspected of containing precancerous or cancer cells or tissues, for example, a tissue biopsy, bone marrow sample or blood sample. Such samples can be, but are not limited to, organs, tissues, tissue fractions and/or cells isolated from an organism such as a mammal, in particular a human. For analysis of B cells, appropriate blood, bone marrow or tissue samples can be employed that contain a suitable number of B cells for analysis. Such samples include, but are not limited to, a tissue section or bone marrow clot section. In a particular embodiment, the tissue section or bone marrow section is formalin fixed, paraffin embedded (FFPE).

The sample or biological specimen can be processed by any of a number of routine methods used for handling biological samples. For example, the cells can be used as isolated or processed, for example, as tissue slices or other tissue specimens, including fixing cells such as with formalin fixed and paraffin embedded (FFPE) tissue, fresh frozen tissue, white blood cells, and the like. Methods for processing tissue or cell samples for *in* situ hybridization are well known to those skilled in the art (see, for example, Stoler, Clinics in Laboratory Medicine 10(1):215-236 (1990); In situ hybridization. A practical approach, Wilkinson, ed., IRL Press, Oxford (1992); Schwarzacher and Heslop-Harrison, Practical in situ hybridization, BIOS Scientific Publishers Ltd, Oxford (2000)). Methods for processing samples for analysis of possible B cell neoplasms are well known in the area of clinical diagnostics and hematopathology (see Knowles, Neoplastic Hematopathology, 2nd edl., Lippincott Williams & Wilkins, Philadelphia PA (2001); Lehmen et al., Am. J. Pathol. 159:2023-2029 (2001); Arber, J. Mol. Diagnsotics 2:178-190 (2000); Plank et al., Am. J. Clin. Pathol. 103:330-337 (1995)).

As disclosed herein, the methods of the invention provide for a convenient assay method to detect multiple target nucleic acids in the same sample. In addition to direct coupling of a label probe with an enzyme or non-enzyme label, in another embodiment an antibody can be used as an intermediary to label the target nucleic acid. Methods have been previously described for detecting two DNA targets simultaneously, such as a two-color chromogenic ISH (CISH) assay (US2011/033176), in which probes against two different genes are conjugated with a hapten such as digoxigenin (DIG) or dinitrophenyl (DNP). Then, anti-DIG and anti-DNP antibodies, or other suitable anti-hapten antibodies, that are conjugated with different enzymes, such as horseradish peroxidase and alkaline phosphatase, are used to generate different color precipitates. While the assay allows visualization of the two targets, the visualization occurs using an antibody to direct the enzyme to the target nucleic acid.

As disclosed herein, by utilizing a label probe comprising nucleic acid to bind to the target nucleic acid, a high level of specificity and selectivity can be achieved by using appropriate *in silico* oligonucleotide design and by selecting and controlling hybridization conditions using well known methods. Such a system can be optimized for selectivity and specificity and minimized for non-specific binding between nucleic acid targets. Furthermore, the methods of the invention provide a system whereby a distinct enzyme or non-enzyme label is targeted to a nucleic acid by way of nucleic acid hybridization, where the enzyme or non-enzyme label is directly coupled to a nucleic acid portion of the label probe. Although the methods of the invention can utilize an antibody as an intermediary to target an enzyme to a nucleic acid, in a particular embodiment, the methods use a label probe comprising a nucleic acid portion that binds to a complementary nucleic acid sequence and an enzyme or non-enzyme label, where direct coupling of the enzyme or non-enzyme label to the nucleic acid portion of the label probe is used.

The invention additionally provides kits comprising components for performing methods of the invention. In one embodiment, the invention provides a kit for detecting immunoglobulin light chain restriction and clonality in human B cells. The kit can contain agents for conducting an RNA *in situ* hybridization assay including at least one probe set which is designed to hybridize to immunoglobulin lambda-like polypeptide 5 (IGLL5) RNA; and a signal amplification system. Generally such a kit would be provided in a suitable container. According to the invention, the kit further comprises at least one probe set which is designed to hybridize to immunoglobulin kappa chain constant region (IGKCR) RNA; and at least one probe set which is designed to hybridize to immunoglobulin lambda chain constant region (IGLCR) RNA. Optionally the kit can further comprise at least one negative control probe set. In a particular embodiment, the kit contains reagents for performing and *in situ* hybridization assay as an RNAscope® assay.

Such kits, in addition to the components described above, can optionally include, for example, label probes, amplifiers, preamplifiers, target probes, and the like. Such components can be designed as for any of the configurations disclosed herein. A kit of the invention can, for example, comprise label probes comprising distinct enzymes and suitable chromogenic or fluorogenic substrates for the respective enzymes, or distinctive non-enzyme labels, for carrying out methods of the invention. The kits can contain non-target-specific components, which can be used for detection of any desired target nucleic acid, with a target specific binding component to be designed separately by the user of the kit, for example probes to IGKCR, IGLCR and/or IGLL5 mRNA. Alternatively, the kit can be designed to contain components that include binding agents for one or more particular target nucleic acids such as IGKCR, IGLCR and/or IGLL5 mRNA. The kit can additionally include instructions for using the components of the kit to carry out methods of the invention.

As described herein, embodiments of the invention can include the use of target probes. Such a configuration and its advantages for increasing sensitivity and decreasing background are described, for example, in U.S. Patent No. 7,709,198, U.S. publications 2008/0038725 and 2009/0081688, and WO 2007/001986 and WO 2007/002006. U.S. Patent No. 7,709,198 and U.S. publications 2008/0038725 and 2009/0081688 additionally describe details for selecting characteristics of the target probes, including target probe pairs, including length, orientation, hybridization conditions, and the like. One skilled in the art can readily identify suitable configurations based on the teachings 7,709,198, U.S. publications 2008/0038725 and 2009/0081688, and WO 2007/001986 and WO 2007/002006. In the description provided below, based on the disclosure herein and the teachings in 7,709,198, U.S. publications 2008/0038725 and 2009/0081688, and WO 2007/001986 and WO 2007/002006, one skilled in the art will understand that the term "label extender," as used in these references and as discussed below, can be used interchangeably with the term "target probe," as described herein and illustrated in the figures.

Briefly, a first general class of embodiments includes methods of detecting two or more nucleic acids of interest. In the methods, a sample comprising or suspected of comprising the nucleic acids of interest, two or more subsets of m label extenders, wherein m is at least two, and a label probe system are provided. Each subset of m label extenders is capable of hybridizing to one of the nucleic acids of interest. According to the invention, the nucleic acid of interest can be IGKCR, IGLCR and IGLL5. The label probe system comprises a label, and a component of the label probe system is capable of hybridizing simultaneously to at least two of the m label extenders in a subset. Each nucleic acid of interest is hybridized to its corresponding subset of m label extenders, and the label probe system is hybridized to the m label extenders. The presence or absence of the label is then detected. Since the label is associated with the nucleic acid(s) of interest via hybridization of the label extenders and label probe system, the presence or absence of the label is correlated with the presence or absence of the nucleic acid(s) of interest and thus in the original sample. The label probe system optionally includes a preamplifier, an amplification multimer, and a label probe, wherein the preamplifier is capable of hybridizing simultaneously to the at least two of the m label extenders and to a plurality of amplification multimers, and wherein the amplification multimer is capable of hybridizing simultaneously to the preamplifier and to a plurality of label probes.

As noted above, a component of the label probe system is capable of hybridizing simultaneously to at least two of the m label extenders in a subset. Typically, the component of the label probe system that hybridizes to the two or more label extenders is an amplification multimer or preamplifier. Thus, in one aspect, the label probe system comprises an amplification multimer or preamplifier, which amplification multimer or preamplifier is capable of hybridizing to the at least two label extenders, and the label probe system is hybridized to the m label extenders at a hybridization temperature, which hybridization temperature is greater than a melting temperature Tₘ of a complex between each individual label extender and the amplification multimer or preamplifier. The hybridization temperature is typically about 5° C or more greater than the Tm, e.g., about 7° C or more, about 10° C or more, about 12° C or more, about 15° C or more, about 17° C or more, or even about 20° C or more greater than the Tm.

Each label extender typically comprises a polynucleotide sequence L-1 that is complementary to a polynucleotide sequence in the corresponding nucleic acid of interest and a polynucleotide sequence L-2 that is complementary to a polynucleotide sequence in the component of the label probe system. In one class of embodiments, the m label extenders in a subset each have L-1 5' of L-2 or each have L-1 3' of L-2. The length of L-2 can vary. For example, sequence L-2 can be 20 nucleotides or less in length, e.g., L-2 can be between 9 and 17 nucleotides in length or between 12 and 15 or between 13 and 15 nucleotides in length.

Another general class of embodiments provides methods of detecting one or more nucleic acids using a label extender configuration. In the methods, a sample comprising or suspected of comprising the nucleic acids of interest, one or more subsets of m label extenders, wherein m is at least two, and a label probe system are provided. Each subset of m label extenders is capable of hybridizing to one of the nucleic acids of interest. The label probe system comprises a label, and a component of the label probe system (e.g., a preamplifier or an amplification multimer) is capable of hybridizing simultaneously to at least two of the m label extenders in a subset. Each label extender comprises a polynucleotide sequence L-1 that is complementary to a polynucleotide sequence in the corresponding nucleic acid of interest and a polynucleotide sequence L-2 that is complementary to a polynucleotide sequence in the component of the label probe system, and the at least two label extenders (e.g., the m label extenders in a subset) each have L-1 5' of L-2 or each have L-1 3' of L-2.

Each nucleic acid of interest is hybridized to its corresponding subset of m label extenders, and the label probe system is hybridized to the m label extenders at a hybridization temperature. The hybridization temperature is greater than a melting temperature Tm of a complex between each individual label extender and the component of the label probe system. Since the label is associated with the nucleic acid(s) of interest via hybridization of the label extenders and label probe system, the presence or absence of the nucleic acid(s) of interest can be determined.

Yet another general class of embodiments provides methods of capturing a label to a first nucleic acid of interest in a multiplex assay in which two or more nucleic acids of interest are to be detected. In the methods, a sample comprising the first nucleic acid of interest and also comprising or suspected of comprising one or more other nucleic acids of interest is provided. A first subset of m label extenders, wherein m is at least two, and a label probe system comprising the label are also provided. The first subset of m label extenders is capable of hybridizing to the first nucleic acid of interest, and a component of the label probe system is capable of hybridizing simultaneously to at least two of the m label extenders in the first subset. The first nucleic acid of interest is hybridized to the first subset of m label extenders, and the label probe system is hybridized to the m label extenders, thereby capturing the label to the first nucleic acid of interest. Essentially all of the features noted for the embodiments above apply to these methods as well, as relevant; for example, with respect to configuration of the label extenders, number of label extenders per subset, composition of the label probe system, type of label, number of nucleic acids of interest, source of the sample and/or nucleic acids, and/or the like.

Yet another general class of embodiments provides methods of capturing a label to a nucleic acid of interest. In the methods, m label extenders, wherein m is at least two, are provided. The m label extenders are capable of hybridizing to the nucleic acid of interest. A label probe system comprising the label is also provided. A component of the label probe system is capable of hybridizing simultaneously to at least two of the m label extenders. Each label extender comprises a polynucleotide sequence L-1 that is complementary to a polynucleotide sequence in the nucleic acid of interest and a polynucleotide sequence L-2 that is complementary to a polynucleotide sequence in the component of the label probe system, and the m label extenders each have L-1 5' of L-2 or wherein the m label extenders each have L-1 3' of L-2. The nucleic acid of interest is hybridized to the m label extenders, and the label probe system is hybridized to the m label extenders at a hybridization temperature, thereby capturing the label to the nucleic acid of interest. Preferably, the hybridization temperature is greater than a melting temperature Tm of a complex between each individual label extender and the component of the label probe system.

A "label extender" or "LE" is a polynucleotide that is capable of hybridizing to a nucleic acid of interest and to a label probe system. The label extender typically has a first polynucleotide sequence L-1, which is complementary to a polynucleotide sequence of the nucleic acid of interest, and a second polynucleotide sequence L-2, which is complementary to a polynucleotide sequence of the label probe system (e.g., L-2 can be complementary to a polynucleotide sequence of an amplification multimer, a preamplifier, a label probe, or the like). The label extender is preferably single-stranded.

A "label probe system" comprises one or more polynucleotides that collectively comprise a label and at least two polynucleotide sequences M-1, each of which is capable of hybridizing to a label extender. The label provides a signal, directly or indirectly. Polynucleotide sequence M-1 is typically complementary to sequence L-2 in the label extenders. The at least two polynucleotide sequences M-1 are optionally identical sequences or different sequences. The label probe system can include a plurality of label probes (e.g., a plurality of identical label probes) and an amplification multimer; it optionally also includes a preamplifier or the like, or optionally includes only label probes, for example.

An "amplification multimer" is a polynucleotide comprising a plurality of polynucleotide sequences M-2, typically (but not necessarily) identical polynucleotide sequences M-2. Polynucleotide sequence M-2 is complementary to a polynucleotide sequence in the label probe. The amplification multimer also includes at least one polynucleotide sequence that is capable of hybridizing to a label extender or to a nucleic acid that hybridizes to the label extender, e.g., a preamplifier. For example, the amplification multimer optionally includes at least one (and generally at least two) polynucleotide sequence(s) M-1, optionally identical sequences M-1; polynucleotide sequence M-1 is typically complementary to polynucleotide sequence L-2 of the label extenders. Similarly, the amplification multimer optionally includes at least one polynucleotide sequence that is complementary to a polynucleotide sequence in a preamplifier. The amplification multimer can be, e.g., a linear or a branched nucleic acid. As noted for all polynucleotides, the amplification multimer can include modified nucleotides and/or nonstandard internucleotide linkages as well as standard deoxyribonucleotides, ribonucleotides, and/or phosphodiester bonds. Suitable amplification multimers are described, for example, in U.S. Pat. No. 5,635,352, U.S. Pat. No. 5,124,246, U.S. Pat. No. 5,710,264, and U.S. Pat. No. 5,849,481.

Signal amplification begins with the binding of the LEs to the target mRNA. An amplification multimer is then typically hybridized to the LEs. The amplification multimer generally has multiple copies of a sequence that is complementary to a label probe and can be a branched-chain nucleic acid; for example, the amplification multimer can be a branched, forked, or comb-like nucleic acid or a linear nucleic acid. A label, for example, an enzyme or non-enzyme label, is covalently attached to each label probe.

In the preceding example, the amplification multimer and the label probes comprise a label probe system. In another example, the label probe system also comprises a preamplifier, e.g., as described in U.S. Pat. No. 5,635,352 and U.S. Pat. No. 5,681,697, which further amplifies the signal from a single target mRNA. In yet another example, the label extenders hybridize directly to the label probes and no amplification multimer or preamplifier is used, so the signal from a single target mRNA molecule is only amplified by the number of distinct label extenders that hybridize to that mRNA.

Among other aspects, the present invention provides multiplex bDNA assays that can be used for simultaneous detection of two or more target nucleic acids. Similarly, one aspect of the present invention provides bDNA assays, singleplex or multiplex, that have reduced background from nonspecific hybridization events.

In general, in the assays of the invention, two or more label extenders are used to capture a single component of the label probe system (e.g., a preamplifier or amplification multimer). The assay temperature and the stability of the complex between a single LE and the component of the label probe system (e.g., the preamplifier or amplification multimer) can be controlled such that binding of a single LE to the component is not sufficient to stably associate the component with a nucleic acid to which the LE is bound, whereas simultaneous binding of two or more LEs to the component can capture it to the nucleic acid. Requiring such cooperative hybridization of multiple LEs for association of the label probe system with the nucleic acid(s) of interest results in high specificity and low background from cross-hybridization of the LEs with other, non-target nucleic acids.

For an assay to achieve high specificity and sensitivity, it preferably has a low background, resulting, e.g., from minimal cross-hybridization. Such low background and minimal cross-hybridization are typically substantially more difficult to achieve in a multiplex assay than a single-plex assay, because the number of potential nonspecific interactions are greatly increased in a multiplex assay due to the increased number of probes used in the assay (e.g., the greater number of CEs and LEs). Requiring multiple simultaneous LE-label probe system component interactions for the capture of the label probe system to a target nucleic acid minimizes the chance that nonspecific capture will occur, even when some nonspecific CE-LE or LE-CP interactions, for example, do occur. This reduction in background through minimization of undesirable cross-hybridization events thus facilitates multiplex detection of the nucleic acids of interest.

The label probe system optionally includes an amplification multimer and a plurality of label probes, wherein the amplification multimer is capable of hybridizing to the label extenders and to a plurality of label probes. In another aspect, the label probe system includes a preamplifier, a plurality of amplification multimers, and a plurality of label probes, wherein the preamplifier hybridizes to the label extenders, and the amplification multimers hybridize to the preamplifier and to the plurality of label probes. As another example, the label probe system can include only label probes, which hybridize directly to the label extenders. In one class of embodiments, the label probe comprises the label, e.g., a covalently attached label. In other embodiments, the label probe is configured to bind a label; for example, a biotinylated label probe can bind to a streptavidin-associated label.

Each label extender typically includes a polynucleotide sequence L-1 that is complementary to a polynucleotide sequence in the corresponding nucleic acid of interest and a polynucleotide sequence L-2 that is complementary to a polynucleotide sequence in the component of the label probe system (e.g., the preamplifier or amplification multimer). It will be evident that the amount of overlap between each individual label extender and the component of the label probe system (i.e., the length of L-2 and M-1) affects the Tm of the complex between the label extender and the component, as does, e.g., the GC base content of sequences L-2 and M-1. Optionally, all the label extenders have the same length sequence L-2 and/or identical polynucleotide sequences L-2. Alternatively, different label extenders can have different length and/or sequence polynucleotide sequences L-2. It will also be evident that the number of label extenders required for stable capture of the component to the nucleic acid of interest depends, in part, on the amount of overlap between the label extenders and the component (i.e., the length of L-2 and M-1).

Stable capture of the component of the label probe system by the at least two label extenders, e.g., while minimizing capture of extraneous nucleic acids, can be achieved, for example, by balancing the number of label extenders that bind to the component, the amount of overlap between the label extenders and the component (the length of L-2 and M-1), and/or the stringency of the conditions under which the label extenders and the component are hybridized. Appropriate combinations of the amount of complementarity between the label extenders and the component of the label probe system, number of label extenders binding to the component, and stringency of hybridization can, for example, be determined experimentally by one of skill in the art. For example, a particular number of label extenders and a particular set of hybridization conditions can be selected, while the number of nucleotides of complementarity between the label extenders and the component is varied until hybridization of the label extenders to a nucleic acid captures the component to the nucleic acid while hybridization of a single label extender does not efficiently capture the component. Stringency can be controlled, for example, by controlling the formamide concentration, chaotropic salt concentration, salt concentration, pH, organic solvent content, and/or hybridization temperature.

As noted, the Tm of any nucleic acid duplex can be directly measured, using techniques well known in the art. For example, a thermal denaturation curve can be obtained for the duplex, the midpoint of which corresponds to the Tm. It will be evident that such denaturation curves can be obtained under conditions having essentially any relevant pH, salt concentration, solvent content, and/or the like.

Typically, the component of the label probe system (e.g., the amplification multimer or preamplifier) is capable of hybridizing simultaneously to two of the m label extenders in a subset, although it optionally hybridizes to three, four, or more of the label extenders. In one class of embodiments, e.g., embodiments in which two (or more) label extenders bind to the component of the label probe system, sequence L-2 is 20 nucleotides or less in length. For example, L-2 can be between 9 and 17 nucleotides in length, e.g., between 12 and 15 nucleotides in length, between 13 and 15 nucleotides in length, or between 13 and 14 nucleotides in length. As noted, m is at least two, and can be at least three, at least five, at least 10, or more. m can be the same or different from subset to subset of label extenders.

The label extenders can be configured in any of a variety ways. For example, the two label extenders that hybridize to the component of the label probe system can assume a cruciform arrangement, with one label extender having L-1 5' of L-2 and the other label extender having L-1 3' of L-2. A configuration in which either the 5' or the 3' end of both label extenders hybridizes to the nucleic acid while the other end binds to the component yields stronger binding of the component to the nucleic acid than does a cruciform arrangement of the label extenders. Thus, in one class of embodiments, the at least two label extenders (e.g., the m label extenders in a subset) each have L-1 5' of L-2 or each have L-1 3' of L-2. For example, L-1, which hybridizes to the nucleic acid of interest, can be at the 5' end of each label extender, while L-2, which hybridizes to the component of the label probe system, is at the 3' end of each label extender (or vice versa). L-1 and L-2 are optionally separated by additional sequence. In one exemplary embodiment, L-1 is located at the 5' end of the label extender and is about 20-30 nucleotides in length, L-2 is located at the 3' end of the label extender and is about 13-14 nucleotides in length, and L-1 and L-2 are separated by a spacer (e.g., 5 Ts).

The methods of the invention as disclosed herein can also be performed with the additional embodiments described below. The steps of such a method can include, for example, providing a sample comprising the cell, which cell comprises or is suspected of comprising one or more nucleic acid targets, for example, a sample comprising a population of B cells; providing for each nucleic acid target a label probe system comprising label; providing for each nucleic acid target a label extender system comprising two or more different label extenders, wherein each of the two or more label extenders comprises a section T complementary to a section on the nucleic acid target and a section L complementary to a section on a component of the label probe system, and wherein the T sections are complementary to non-overlapping regions of the nucleic acid target, and the L sections are complementary to non-overlapping regions of the component of the label probe system; hybridizing, in the cell, the label extenders system to the nucleic acid target, when present in the cell, thereby providing hybridization of two or more different label extenders to a single copy of the nucleic acid target molecule; capturing, in the cell and in the presence of cellular non-target nucleic acids, the label probe system to the label extender system hybridized to the nucleic acid target molecule, thereby capturing the label to the nucleic acid target, wherein the capturing occurs by simultaneously hybridizing the at least two different label extenders to a single molecule of the component of the label probe system that is complementary to the label extenders; and detecting a signal from the label.

An embodiment can include hybridizing the at least two different label extenders to the single molecule of the component of the label probe system that is complementary to the label extenders is performed at a hybridization temperature that is greater than a melting temperature Tm of a complex between each individual label extender and the component of the label probe system, for example, the label probe, amplifier or preamplifier. In another embodiment, the at least two different label extenders hybridize to adjacent sections on the nucleic acid target. Also, hybridizing the two or more label extenders to the single molecule of the nucleic acid target can be performed at a hybridization temperature that is greater than a melting temperature Tm of a complex between each individual label extender and the nucleic acid target. Another embodiment can further comprise providing one or more blocking probes capable of hybridizing to regions of the nucleic acid target not occupied by the label extenders. In still another embodiment, each hybridizing or capturing step is accomplished for all the nucleic acid targets at the same time. In a further embodiment, the one or more nucleic acid targets can comprise a reference nucleic acid, and wherein the method comprises normalizing the signal of the one or more nucleic acid targets to the signal of the reference nucleic acid. Still a further embodiment can comprise the step of correlating the intensity of the signal of each nucleic acid target with a quantity of the corresponding nucleic acid target present in the cell. The sample comprising the cell can be derived from a bodily fluid, from blood or a tissue section, as disclosed herein. The cell can be in suspension during the hybridizing, capturing, and/or detecting steps but is typically performed as an *in situ* hybridization assay.

In a particular embodiment, each label extender is in a "Z" configuration, wherein the Z configuration comprises the T section of the label extender at the 5' end and the L section of the label extender at the 3' end, or the T section of the label extender at the 3' end and the L section of the label extender at the 5' end. In another embodiment, the label probe system comprises (A) (i) a label probe comprising one or more labels, or (B) (i) one or more label probes and (ii) an amplifier capable of hybridizing to one or more of the label probes, or (C) (i) one or more label probes, (ii) one or more amplifiers, and (iii) a preamplifier capable of hybridizing to one or more of the amplifiers. In a further embodiment, the label is captured to the nucleic acid target molecule by hybridization of the label probe to the at least two different label extenders hybridized to the nucleic acid target molecule. In still a further embodiment, the label is captured to the nucleic acid target molecule by hybridization of the one or more label probes to the amplifier molecule hybridized to the at least two different label extenders hybridized to the nucleic acid target molecule. In yet another embodiment, the label is captured to the nucleic acid target molecule by hybridization of the one or more label probes to the one or more amplifiers hybridized to the preamplifier molecule hybridized to the at least two different label extenders hybridized to the nucleic acid target molecule. In a still further embodiment, a plurality of label probe systems are provided for each target nucleic acid and wherein two or more label probe systems are captured to the target nucleic acid by using the label extender system comprising two or more different label extenders and wherein each label extender in the label extender system comprises a T section that is complementary to non-overlapping regions of the nucleic acid target. In yet another embodiment, three or more label probe systems are captured to the target nucleic acid by the label extender system.

The methods of the invention can additionally include further aspects as described below. For example, the methods can further include a method of detecting one or more nucleic acids of interest, the method comprising: a) providing a sample comprising or suspected of comprising the one or more nucleic acids of interest; b) labeling those nucleic acids of interest present in the sample using the methods disclosed herein; c) providing one or more subsets of m label extenders, wherein m is at least two, wherein each subset of m label extenders is capable of hybridizing to one of the nucleic acids of interest; d) providing a label probe system comprising a label, wherein a component of the label probe system is capable of hybridizing simultaneously to at least two of the m label extenders in a subset, wherein each of the at least two label extenders comprises a polynucleotide sequence L-1 that is complementary to a polynucleotide sequence in the corresponding nucleic acid of interest and comprises a polynucleotide sequence L-2 that is complementary to a polynucleotide sequence in the component of the label probe system, wherein the at least two label extenders all have L-1 5' of L-2 or all have L-1 3' of L-2, and wherein the m label extenders in the subset are complementary to nonoverlapping sequences in the corresponding nucleic acid of interest; e) hybridizing any nucleic acid of interest captured to its corresponding subset of m label extenders; f) hybridizing the label probe system to the m label extenders at a hybridization temperature, which hybridization temperature is greater than a melting temperature Tm of a complex between each individual label extender and the component of the label probe system; and g) detecting the presence or absence of the label on the target nucleic acid.

In a particular embodiment, the label probe system comprises a preamplifier, an amplification multimer and a label probe; wherein the preamplifier is capable of hybridizing simultaneously to the at least two of the m label extenders and to a plurality of amplification multimers: wherein the amplification multimer is capable of hybridizing simultaneously to the preamplifier and to a plurality of label probes; and wherein the label probe comprises the label. In another embodiment, one or more nucleic acids of interest comprise two or more different nucleic acids of interest, which different nucleic acids of interest are different nucleic acid molecules, and wherein the one or more subsets of m label extenders comprise two or more different subsets of m label extenders. In a particular embodiment, the label probe system comprises an amplification multimer or preamplifier, which amplification multimer or preamplifier is capable of hybridizing to the at least two label extenders.

In an embodiment, the hybridization temperature is about 5° C or more greater than the Tm of a complex between each individual label extender and the component of the label probe system. Alternatively, the hybridization temperature is about 7° C or more, about 10° C or more, about 12° C or more, about 15° C or more, about 17° C or more, or about 20° C or more greater than the Tm of a complex between each individual label extender and the component of the label probe system. In another embodiment, the polynucleotide sequence L-2 is 20 nucleotides or less in length. Alternatively, L-2 is between 9 and 17 nucleotides in length. Alternatively, L-2 is between 13 and 15 nucleotides in length.

In another embodiment, each of the at least two label extenders has L-1 at its 5' end and L-2 at its 3' end. In still another embodiment, each of the at least two label extenders has L-1 at its 3' end and L-2 at its 5' end. In still another embodiment, the m label extenders in a subset all have L-1 5' of L-2 or all have L-1 3' of L-2. In another embodiment, the label probe system comprises an amplification multimer and a label probe; wherein the amplification multimer is capable of hybridizing simultaneously to the at least two of the m label extenders and to a plurality of label probes; and wherein the label probe comprises the label. In another embodiment, the label probe system comprises a label probe; wherein the label probe is capable of hybridizing simultaneously to the at least two of the m label extenders; and wherein the label probe comprises the label. In another embodiment, the component of the label probe system is capable of hybridizing simultaneously to two of the m label extenders in a subset.

A method of the invention can further involve capturing a label to a nucleic acid of interest, by a) providing m label extenders, wherein in is at least two, wherein the m label extenders are capable of hybridizing to nonoverlapping sequences in the nucleic acid of interest; b) providing a label probe system comprising the label, wherein a component of the label probe system is capable of hybridizing simultaneously to at least two of the m label extenders, wherein each of the at least two label extenders comprises a polynucleotide sequence L-1 that is complementary to a polynucleotide sequence in the nucleic acid of interest and comprises a polynucleotide sequence L-2 that is complementary to a polynucleotide sequence in the component of the label probe system, and wherein the at least two label extenders all have L-1 5' of L-2 or all have L-1 3' of L-2; c) hybridizing the nucleic acid of interest to the m label extenders; and d) hybridizing the label probe system to the in label extenders at a hybridization temperature, which hybridization temperature is greater than a melting temperature Tm of a complex between each individual label extender and the component of the label probe system, thereby capturing the label to the nucleic acid of interest.

In an embodiment, the m label extenders all have L-1 5' of L-2 or all have L-1 3' of L-2. In an embodiment, the label probe system comprises an amplification multimer or preamplifier, which amplification multimer or preamplifier is capable of hybridizing to at least two of the m label extenders. In an embodiment, the label probe system comprises a preamplifier, an amplification multimer and a label probe; wherein the preamplifier is capable of hybridizing simultaneously to at least two of the m label extenders and to a plurality of amplification multimers; wherein the amplification multimer is capable of hybridizing simultaneously to the preamplifier and to a plurality of label probes; and wherein the label probe comprises the label. In an embodiment, the label probe system comprises an amplification multimer and a label probe; wherein the amplification multimer is capable of hybridizing simultaneously to the at least two of the m label extenders and to a plurality of label probes; and wherein the label probe comprises the label. In an embodiment, the label probe system comprises a label probe; wherein the label probe is capable of hybridizing simultaneously to the at least two of the m label extenders; and wherein the label probe comprises the label. In an embodiment, the component of the label probe system is capable of hybridizing simultaneously to two of the m label extenders. In an embodiment, the hybridization temperature is about 5° C or more greater than the Tm of a complex between each individual label extender and the component of the label probe system. In an embodiment, the hybridization temperature is about 7° C or more, about 10° C or more, about 12° C or more, about 15° C or more, about 17° C or more, or about 20° C or more greater than the Tm of a complex between each individual label extender and the component of the label probe system. In an embodiment, the polynucleotide sequence L-2 is 20 nucleotides or less in length. In an embodiment, L-2 is between 9 and 17 nucleotides in length. In an embodiment, L-2 is between 13 and 15 nucleotides in length. In an embodiment, each of the at least two label extenders has L-1 at its 5' end and L-2 at its 3' end. In an embodiment, each of the at least two label extenders has L-1 at its 3' end and L-2 at its 5' end.

A further embodiment can include a method of detecting one or more nucleic acids of interest, by a) providing a sample comprising the one or more nucleic acids of interest; b) labeling the nucleic acids of interest using the methods disclosed herein; c) providing a label probe system comprising a label; d) for each of the nucleic acids of interest, providing a subset of m label extenders, wherein m is at least two, wherein the subset of m label extenders is capable of hybridizing to that nucleic acid of interest, wherein a component of the label probe system is capable of hybridizing simultaneously to at least two of the m label extenders in the subset, wherein each of the at least two label extenders comprises a polynucleotide sequence L-1 that is complementary to a polynucleotide sequence in the nucleic acid of interest and comprises a polynucleotide sequence L-2 that is complementary to a polynucleotide sequence in the component of the label probe system, and wherein the at least two label extenders all have L-1 5' of L-2 or all have L-1 3' of L-2; e) hybridizing each nucleic acid of interest to its corresponding subset of m label extenders; f) hybridizing the label probe system to the label extenders at a hybridization temperature, which hybridization temperature is greater than a melting temperature Tm of a complex between each individual label extender and the component of the label probe system, whereby, for each of the nucleic acids of interest, a single copy of each of the at least two label extenders hybridizes simultaneously to a single copy of the nucleic acid of interest and to a single copy of the component of the label probe system; and g) detecting the presence or absence of the label on the target nucleic acid.

In yet another embodiment, a method can include capturing a label to a nucleic acid of interest, by: a) providing m label extenders, wherein m is at least two, wherein the m label extenders are capable of hybridizing to the nucleic acid of interest; b) providing a label probe system comprising the label, wherein a component of the label probe system is capable of hybridizing simultaneously to at least two of the m label extenders, wherein each of the at least two label extenders comprises a polynucleotide sequence L-1 that is complementary to a polynucleotide sequence in the nucleic acid of interest and comprises a polynucleotide sequence L-2 that is complementary to a polynucleotide sequence in the component of the label probe system, and wherein the at least two label extenders all have L-1 5' of L-2 or all have L-1 3' of L-2; c) hybridizing the nucleic acid of interest to the m label extenders; and d) hybridizing the label probe system to the m label extenders at a hybridization temperature, which hybridization temperature is greater than a melting temperature Tm of a complex between each individual label extender and the component of the label probe system, whereby a single copy of each of the at least two label extenders hybridizes simultaneously to a single copy of the nucleic acid of interest and to a single copy of the component of the label probe system, thereby capturing the label to the nucleic acid of interest.

It is understood that modifications which do not substantially affect the activity of the various embodiments of this invention are also provided within the definition of the invention provided herein. Accordingly, the following examples are intended to illustrate but not limit the present invention.

### EXAMPLE

### Detection of Immunoglobulin Light Chain Restriction in B Cells Using In situ Hybridization

This example describes *in situ* hybridization assays to detect immunoglobulin light chain restriction in B cells.

An RNAscope® assay (Advanced Cell Diagnostics) to detect Ig kappa and lambda light chain mRNA for detecting LCR in samples of both benign proliferative lymphoid disorders and B cell neoplasms. RNAscope® probes were designed to target IGKC (immunoglobulin kappa chain constant region) and IGLC (immunoglobulin lambda chain constant region) genes, which encode the constant region of kappa and lambda light chain, respectively. The RNAscope® assay is capable of detecting very low levels of kappa/lambda transcripts (<10 copies/cell) in B cells in human tonsil (Wang et al., 2012). Described below is the application of an RNAscope® assay and development of an interpretation algorithm for LCR detection in B cell proliferative diseases. When tissue sections of a wide variety of lymphoproliferative diseases that had been evaluated for B cell lymphomas were stained with kappa and lambda probes, five patterns of expression were detected. These experiments and results are described below in more detail.

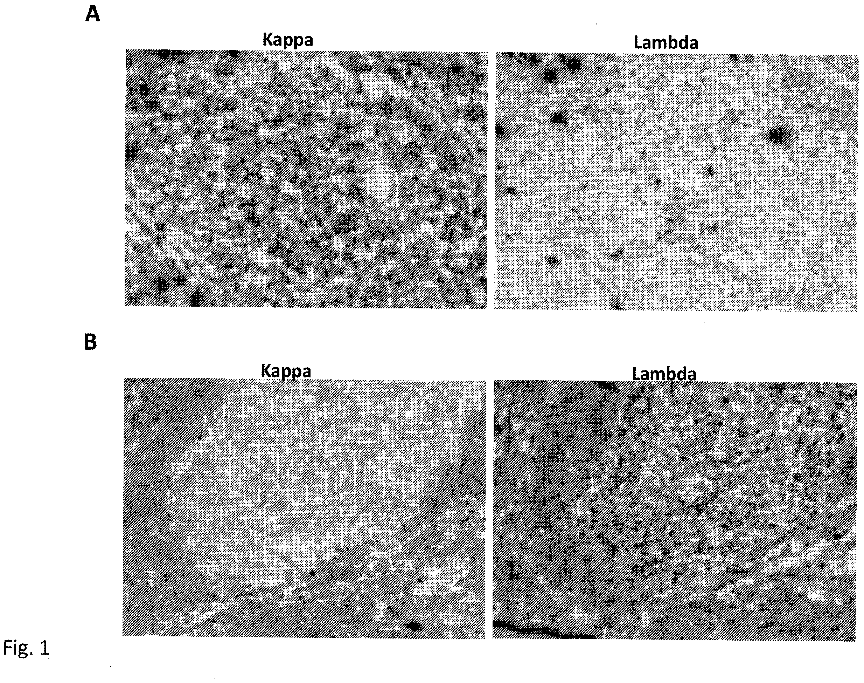

Figures 1A and 1B show examples of clear restricted light chain expression in kappa-restricted (Pattern 1, Figure 1A, left panel) and lambda-restricted (Pattern 2, Figure 1B, right panel) lymphomas, where one of the two light chains was expressed in the tumor cells. Strong lambda staining in some cells in Figure 1A were from nonmalignant plasma cells, which produce high levels of light chains. Kappa staining outside the tumor region in Figure 1B was from normal B lymphocytes. This type of kappa-restricted (Pattern 1) or lambda-restricted (Pattern 2) expression pattern has traditionally been used as a diagnostic for B cell lymphoma in a sample displaying a kappa restricted or lambda restricted clonal phenotype.

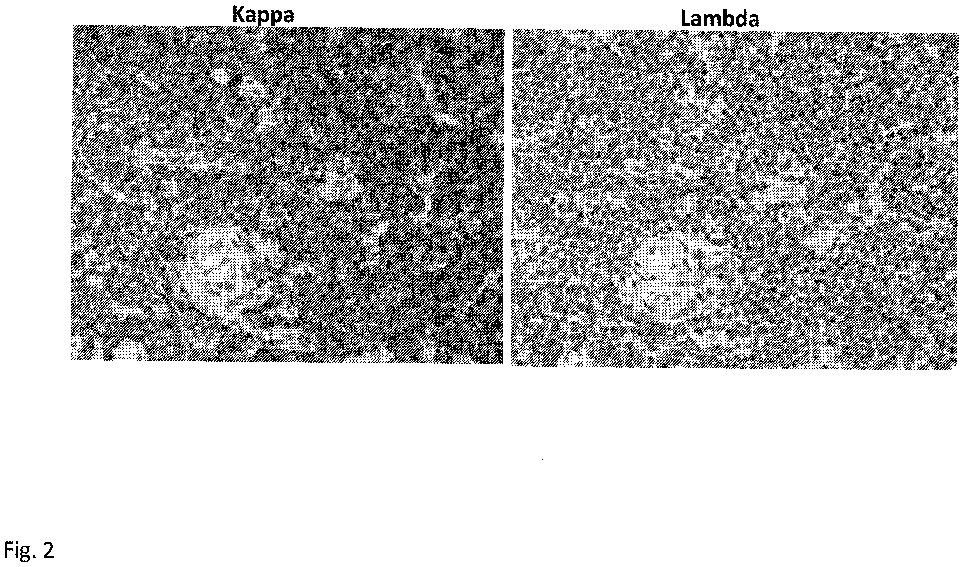

Figure 2 shows an example of a third staining pattern. In this kappa-restricted lymphoma case as determined by flow cytometry, positive lambda staining cells were also detected in the same region of kappa expression, making it difficult to conclude whether these cells were kappa-restricted.

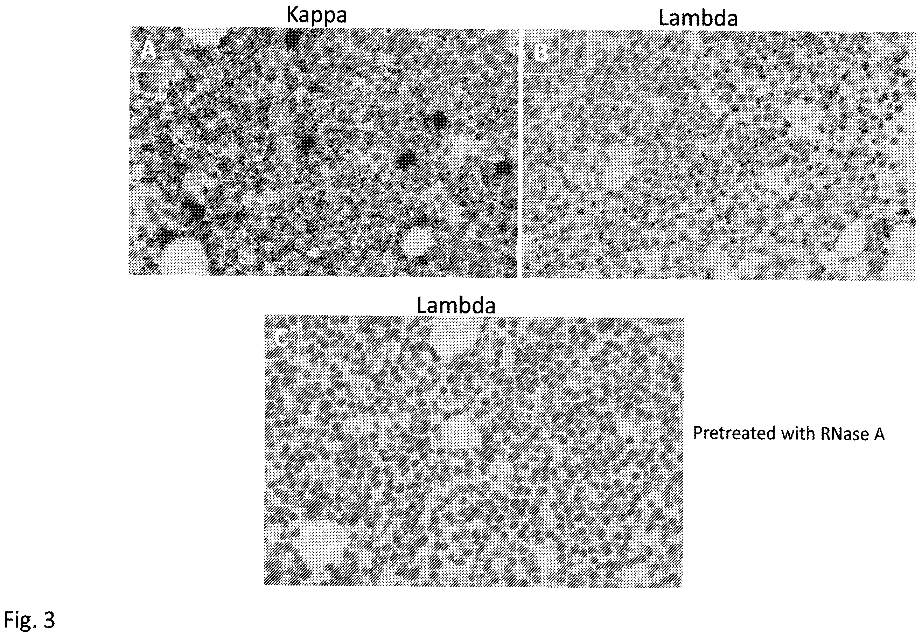

Figures 3A and 3B show another example of the same pattern, where lamda staining cells were detected in the same region as kappa staining cells. An experiment was performed to determine whether the lambda signals were derived from probes hybridizing to genomic DNA at the 7 IGLC loci. An adjacent tissue section was pretreated with RNase and then stained with the lambda probe. RNase treatment eliminated the lambda signals, demonstrating that the signals detected by the lambda probe originated from RNA.

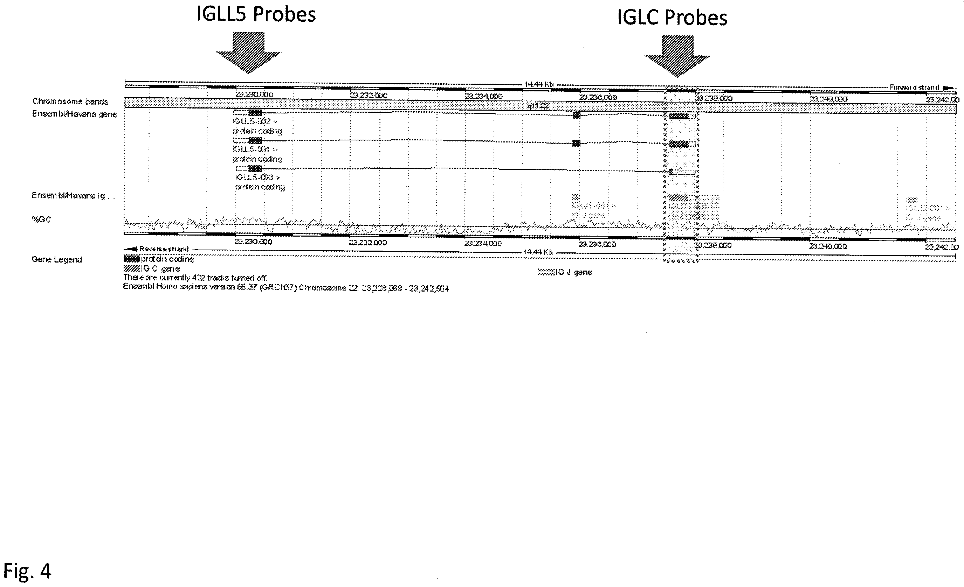

The genomic map of IGLC genes was examined. It was noted that the IGLC1 sequence is also the third exon of a gene called IGLL5 (immunoglobulin lambda-like polypeptide 5; NCBI ENTREZ Gene ID 100423062). Therefore, the lambda probe targeting IGLC can detect expression either from IGLC1 or from IGLL5. IGLL5 has been shown to be expressed in B cells from the unrearranged IGLC locus (Evans & Hollis, J. Experimental Med., 173:305-311(1991); Guglielmi & Davi, Eur. J. Immunol., 21:501-508 (1991)). Figure 4 shows a schematic diagram of a portion of human chromosome 22 in a region encoding the Ig lambda light chain. As depicted in the diagram of Figure 4, *IGLL5* transcripts share sequences with canonical immunoglobulin light chain. With regard to IGLL5, it can be characterized as follows: (1) *IGLL5* expression does not require gene rearrangement; (2) *IGLL5* exon 1 and part of exon 2 are deleted during Ig lambda V-J rearrangement; (3) *IGLL5* is expressed only in kappa-producing B cells and at highly variable levels; and (4) the function of *IGLL5* and whether IGLL5 protein is produced are unknown. The exon-intron structure of IGLL5 can be found at ncbi.nlm.nih.gov/gene/100423062 (NCBI ENTREZ Gene ID 100423062). The sequences of two alternatively spliced IGLL5 mRNAs can be found in GenBank accessions NM_001178126.1 and NM_001256296.1.

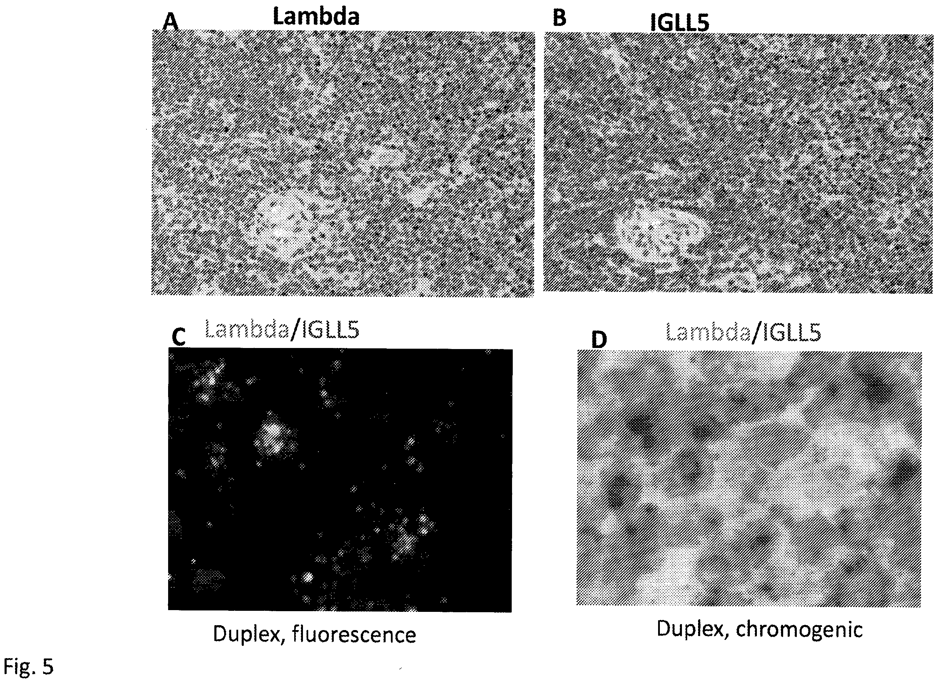

To show that the lambda signals detected in the third pattern of kappa and lambda expression (Figures 2 and 3) might come from IGLL5, IGLL5-specific probes targeting exon 1 of IGLL5 were designed (Figure 4). Figures 5A-5D show staining of tissue sections with lambda and IGLL5 probes. Figures 5A and 5B show staining of adjacent tissue sections with lambda and IGLL5 probes, respectively. Figures 5C and 5D show a duplex assay using lambda and IGLL5 probes detected by fluorescence or a chromogenic assay, respectively. When adjacent sections were stained with lambda and IGLL5 probes, the signal patterns were nearly identical (Figures 5A and 5B), as expected if both IGLL5 and lambda probes were detecting the same IGLL5 transcripts. To further confirm this, a duplex hybridization assay was performed with lambda and IGLL5 probes. Fluorescence detection of IGLL5 (red; Alexa Fluor™ 647) and lambda (green; Alexa Fluor™ 488) was performed on the sample (Figure 5C). Both signals were localized in the same cells, and many signals overlapped to produce yellow dots in the merged image, indicating that the same transcripts were detected by both probes. The non-overlapping green and red signals in these cells were likely due to incomplete hybridization resulting in only one of the two probes hybridizing successfully to their targets. Similar results were observed in a duplex assay performed with chromogenic staining (Figure 5D), where lambda was stained green (HRP-Green; 42 life sciences GmbH & Co.; Bremerhaven Germany) and IGLL5 was stained red (Fast Red). These experiments together with the known literature regarding IGLL5 led to the conclusion that the third pattern, where kappa and what appeared to be lambda co-expression, was due to IGLL5 expression.

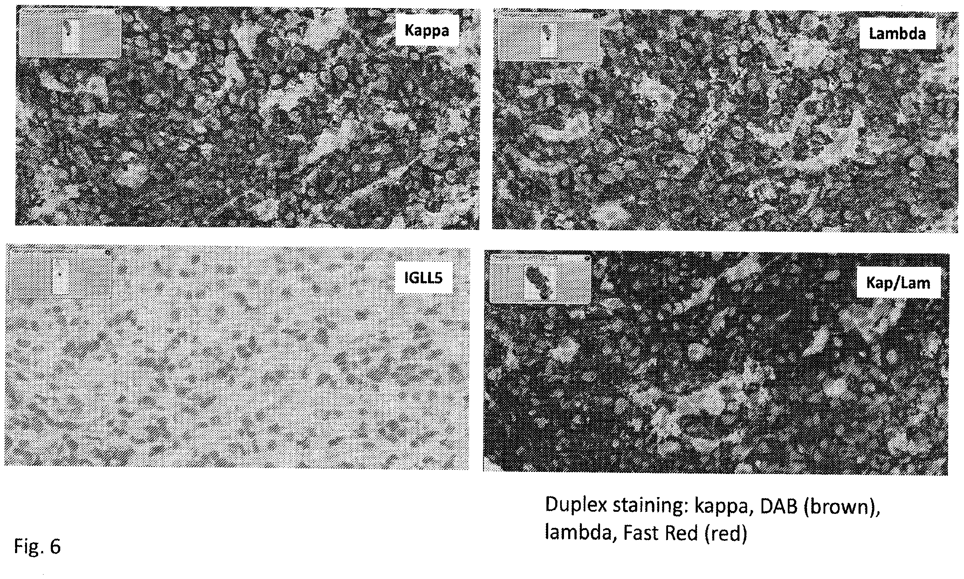

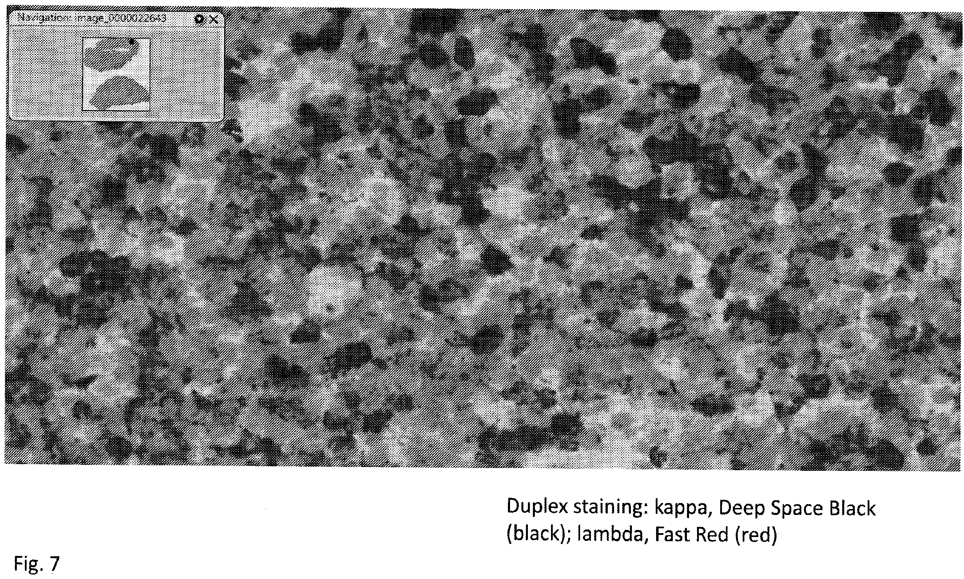

In another experiment, three probes, kappa, lambda and IGLL5, were used to analyze B cell lymphomas. Interestingly, a fourth pattern of expression was observed in some samples (Figure 6). In this pattern, kappa and lambda signals were present in the same cells as demonstrated by duplex staining (bottom right panel in Figure 6), but little or no IGLL5 signals were detected (bottom left panel in Figure 6). Therefore, the observed lambda signals were not from IGLL5. Thus, in these cells, both kappa and lambda light chains were expressed and were thus non-restricted. This non-restricted pattern still marked a clonal population of cells since all cells showed the same non-restricted pattern. This non-restricted clonal pattern is different from the non-restricted polyclonal pattern (Pattern 5) shown in Figure 7. In the latter case, kappa and lambda signals were seen in different cells, which is typical of benign lymphoproliferative diseases. Dual light chain expression in B cell lymphomas has been described before based on flow cytometry detecting light chain proteins ((Xu, Arch. Pathol. Lab. Med. 130:853-856 (2006)). The results described here and shown in Figure 6 represent the first known demonstration of dual light chain expression at the mRNA level.

The results described above have led to the development of a schematic or algorithm that provides light chain restriction determination based on patterns of kappa, lambda and IGLL5 mRNA expression (see Figure 8). One implementation of the schematic or algorithm is shown in Figure 8. Due to the observed multiple patterns of kappa, lambda and IGLL5 mRNA expression, as described above, a 2-slide two-color duplex assay was implemented as shown in Figure 8. In the exemplified embodiment, one slide is stained with kappa (black or another suitable and distinguishable detection system) and lambda (red or other suitable and distinguishable detection system) probes, and the other slide is stained with dapB (black or another suitable and distinguishable detection system) and IGLL5 (red or another suitable and distinguishable detection system). The bacterial gene dapB is used as negative control. Patterns of 1 (kappa-restricted), 2 (lambda-restricted) and 5 (non-restricted, polyclonal) can be easily interpreted based on the kappa/lambda slide alone. However, if both kappa and lambda signals are detected in the same cells, the dapB/IGLL5 slide can be examined to distinguish between patterns 4 and 5. If IGLL5 signals are similar to or even greater than the lambda probe signals, it indicates kappa-restriction. If IGLL5 signals are absent or much less than the lambda probe signals, it indicates a clonal, non-restricted pattern. Therefore, by analyzing these 3 genes, namely kappa and lambda expression and optionally IGLL5 expression, and applying the interpretation system and algorithm depicted in Figure 8, LCR and clonality can be unambigously determined.

These results provide a highly sensitive, accurate and rapid assay to determine light chain restriction, particularly in cells having low kappa and/or lambda expression, as well as identifying clonal B cell populations that exhibit apparent or actual co-expression of kappa and lambda. The assay can therefore be used to identify clonal and likely neoplastic samples that would otherwise have been identified as negative or indeterminate with respect to light chain restriction.

## Claims

1. A method for detecting immunoglobulin light chain restriction and clonality in B cells, the method comprising:
(a) conducting a duplex *in situ* hybridization assay on a sample of B cells from a subject using
at least one probe set which is designed to specifically hybridize to immunoglobulin kappa chain constant region (IGKCR) RNA;
at least one probe set which is designed to specifically hybridize to immunoglobulin lambda chain constant region (IGLCR) RNA; and
at least one probe set which is designed to specifically hybridize to immunoglobulin lambda-like polypeptide 5 (IGLL5) RNA;
(b) detecting signal associated with hybridized IGKCR probe, signal associated with hybridized IGLCR probe and signal associated with hybridized IGLL5 probe in a population of B cells in the sample; and
(c) determining a pattern of signal associated with hybridized IGKCR probe, hybridized IGLCR probe and hybridized IGLL5 probe within individual cells in the B cell population,
wherein
the pattern, where both signal associated with IGKCR probe and signal associated with IGLCR probe are present in the same B cell, and where the level of signal associated with hybridized IGLL5 probe is greater than or equal to the level of signal associated with hybridized IGLCR probe, indicates kappa restriction; or
the pattern, where both signal associated with IGKCR probe and signal associated with IGLCR probe are present in the same B cell, and where the level of signal associated with hybridized IGLL5 probe is less than the level of signal associated with hybridized IGLCR probe, indicates a clonal, non-restricted sample.

2. The method of claim 1, wherein the *in situ* hybridization assay is conducted as singleplex reactions on serial sections.

3. The method of claim 1, wherein hybridization of the IGKCR probe set and the IGLCR probe set are hybridized in the same *in situ* hybridization assay.

4. The method of claim 3, wherein hybridization of the IGLL5 probe set is in a separate *in situ* hybridization assay from the assay in which the IGKCR probe set and the IGLCR probe set are hybridized.

5. The method of any one of claims 1-4, further comprising using at least one negative control probe set.

6. The method of any one of claims 1-5, wherein the sample is a tissue section or a bone marrow clot section.

7. The method of claim 6, wherein the tissue section or bone marrow clot section is formalin fixed, paraffin embedded.

8. The method of any one of claims 1-7, wherein the subject is human.

9. A kit for detecting immunoglobulin light chain restriction and clonality in human B cells comprising agents for conducting an RNA *in situ* hybridization assay including:
at least one probe set which is designed to hybridize to immunoglobulin lambda-like polypeptide 5 (IGLL5) RNA;
at least one probe set which is designed to hybridize to immunoglobulin kappa chain constant region (IGKCR) RNA;
at least one probe set which is designed to hybridize to immunoglobulin lambda chain constant region (IGLCR) RNA; and
a signal amplification system.

10. The kit of claim 9, further comprising at least one negative control probe set.

## Patentansprüche

1. Verfahren zum Detektieren der Restriktion der leichten Immunglobulinkette und der Klonalität in B-Zellen, das Verfahren umfassend:
(a) Durchführen eines *in situ* Doppel-Hybridisationstests einer Probe von B-Zellen aus einem Subjekt unter Verwendung von
mindestens einem Sondensatz, welcher konzipiert ist, spezifisch mit der RNA der konstanten Region der Immunglobulinkette Kappa (IGKCR) zu reagieren;
mindestens einem Sondensatz, welcher konzipiert ist, spezifisch mit der RNA der konstanten Region der Immunglobulinkette Lambda (IGLCR) zu hybridisieren; und
mindestens einem Sondensatz, welcher konzipiert ist, spezifisch mit der RNA des Lambda-ähnlichen Immunglobulin-Polypeptids 5 (IGLL5) zu reagieren;
(b) Detektieren des Signals, welches mit der hybridisierten IGKCR-Sonde assoziiert ist, des Signals, welches mit der hybridisierten LGLCR-Sonde assoziiert ist und des Signals, welches mit der hybridisierten IGLL5-Sonde assoziiert ist, in einer Population von B-Zellen in der Probe; und
(c) Bestimmung eines Signalmusters, welches mit der hybridisierten IGKCR-Sonde, mit der hybridisierten IGLCR-Sonde und mit der hybridisierten IGLL5-Sonde assoziiert ist, in einzelnen Zellen in der B-Zell Population,
wobei
das Muster, bei dem sowohl das Signal, welches mit der IGKCR-Sonde assoziiert ist als auch das Signal, welches mit der IGLCR-Sonde assoziiert ist, in den selben B-Zellen vorliegt und bei dem die Stärke des Signals, welches mit der hybridisierten IGLL5-Sonde assoziiert ist, größer oder gleich der Stärke des Signals ist, welches mit der hybridisierten IGLCR-Sonde assoziiert ist, Kappa-Restriktion anzeigt; oder
das Muster, bei dem sowohl das Signal, welches mit der IGKCR-Sonde assoziiert ist als auch das Signal, welches mit der IGLCR-Sonde assoziiert ist, in den selben B-Zellen vorliegt und bei dem die Stärke des Signals, welches mit der hybridisierten IGLL5-Sonde assoziiert ist, niedriger ist als die Stärke des Signals, welches mit der hybridisierten IGLCR-Sonde assoziiert ist, eine klonale, nicht restringierte Probe anzeigt.

2. Verfahren nach Anspruch 1, wobei der *in situ* Hybridisationstest als Singleplex-Reaktionen auf Serienschnitten durchgeführt wird.

3. Verfahren nach Anspruch 1, wobei die Hybridisation des IGKCR-Sondensatzes und des IGLCR-Sondensatzes im selben *in situ* Hybridisationstest hybridisiert werden.

4. Verfahren nach Anspruch 3, wobei die Hybridisation des IGLL5-Sondensatzes in einem separaten *in situ* Hybridisationstest erfolgt von dem Test, in dem der IGKCR-Sondensatz und der IGLCR-Sondensatz hybridisiert werden.

5. Verfahren nach einem der Ansprüche 1 bis 4, welches ferner die Verwendung von mindestens einem Negativ-Kontrollsondensatzes umfasst.

6. Verfahren nach einem der Ansprüche 1 bis 5, wobei die Probe ein Gewebeschnitt oder ein Knochenmarkgerinsel-Schnitt ist.

7. Verfahren nach Anspruch 6, wobei der Gewebeschnitt oder der Knochenmarkgerinsel-Schnitt mit Formalin fixiert oder in Paraffin eingebettet ist.

8. Verfahren nach einem der Ansprüche 1 bis 7, wobei das Subjekt ein Mensch ist.

9. Kit zum Detektieren von Restriktion der leichten Immunglobulinketten und der Klonalität in menschlichen B-Zellen, welches Agentien zum Durchführen eines *in situ* RNA-Hybridisationstests umfasst, aufweisend:
mindestens einen Sondensatz, welcher konzipiert ist, an die RNA des Lambda-ähnlichen Immunglobulin-Polypeptids 5 (IGLL5)-zu hybridisieren;
mindestens einen Sondensatz, welcher konzipiert ist, an die RNA der Immunglobulinkette Kappa der konstanten Region (IGKCR) zu hybridisieren;
mindestens einen Sondensatz, welcher konzipiert ist, an die RNA der Immunglobulinkette Lambda der Konstanten Region (IGLCR) zu hybridisieren; und
ein Signal-Amplifikationssystem.

10. Kit nach Anspruch 9, welches ferner mindestens einen Negativkontroll-Sondensatz umfasst.

## Revendications

1. Procédé pour détecter une restriction d'une chaîne légère d'immunoglobuline et la clonalité dans les cellules B, le procédé comprenant:
(a) effectuer un essai d'hybridation duplex *in situ* sur un échantillon de cellules B d'un sujet en utilisant
au moins un jeu de sondes qui est conçu pour s'hybrider spécifiquement à l'ARN d'une région constante de chaîne Kappa d'immunoglobuline (IGKCR);
au moins un jeu de sondes qui est conçu pour s'hybrider spécifiquement à l'ARN d'une région constante de chaîne lambda d'immunoglobuline (IGLCR); et
au moins un jeu de sondes qui est conçu pour s'hybrider spécifiquement à l'ARN d'un polypeptide de type immunoglobuline lambda 5 (IGLL5);
(b) détecter un signal associé à la sonde IGKCR hybridée, un signal associé à la sonde IGLCR hybridée et un signal associé à la sonde IGLL5 hybridée dans une population de cellules B dans l'échantillon; et
(c) déterminer un motif de signal associé à une sonde IGKCR hybridée, une sonde IGLCR hybridée et une sonde IGLL5 hybridée dans des cellules individuelles dans la population de cellules B,
dans lequel
le motif, où à la fois un signal associé à la sonde IGKCR et un signal associé à la sonde IGLCR sont présents dans la même cellule B, et où le niveau de signal associé à la sonde IGLL5 hybridée est supérieur ou égal au niveau de signal associé à la sonde IGLCR hybridée, indique une restriction kappa; ou
le motif, où à la fois un signal associé à la sonde IGKCR et un signal associé à la sonde IGLCR sont présents dans la même cellule B, et où le niveau de signal associé à la sonde IGLL5 hybridée est inférieur au niveau de signal associé à la sonde IGLCR hybridée, indique un échantillon clonal, et non restreint.

2. Procédé selon la revendication 1, dans lequel l'essai d'hybridation *in situ* est réalisé sous forme de réactions uniques sur des sections en série.

3. Procédé selon la revendication 1, dans lequel l'hybridation du jeu de sondes IGKCR et du jeu de sondes IGLCR sont hybridés dans le même essai d'hybridation *in situ.*

4. Procédé selon la revendication 3, dans lequel l'hybridation du jeu de sondes IGLL5 est réalisée dans un essai d'hybridation *in situ* séparé à partir de l'essai dans lequel le jeu de sondes IGKCR et le jeu de sondes IGLCR sont hybridés.

5. Procédé selon l'une quelconque des revendications 1 à 4, comprenant en outre l'utilisation d'au moins un jeu de sondes de contrôle négatif.

6. Procédé selon l'une quelconque des revendications 1 à 5, dans lequel l'échantillon est une section de tissu ou une section de caillot de moelle osseuse.

7. Procédé selon la revendication 6, dans lequel la section de tissu ou la section de caillot de moelle osseuse est fixée à la formaline, incluse dans la paraffine.

8. Procédé selon l'une quelconque des revendications 1 à 7, dans lequel le sujet est humain.

9. Kit pour détecter la restriction d'une chaîne légère d'immunoglobuline et la clonalité dans les cellules B humaines comprenant des agents pour effectuer un dosage d'hybridation *in situ* d'ARN incluant:
au moins un jeu de sondes qui est conçu pour s'hybrider à l'ARN du polypeptide de type immunoglobuline lambda 5 (IGLL5);
au moins un ensemble de sonde qui est conçu pour s'hybrider spécifiquement à l'ARN d'une région constante de chaîne Kappa d'immunoglobuline (IGKCR);
au moins un ensemble de sonde conçu pour s'hybrider spécifiquement à l'ARN d'une région constante de chaîne lambda d'immunoglobuline (IGLCR); et
un système d'amplification du signal.

10. Kit selon la revendication 9, comprenant en outre l'utilisation d'au moins un jeu de sondes de contrôle négatif.
